(19) Europäisches Patentamt / European Patent Office / Office européen des brevets

(11) **EP 1 368 372 B1**

(12) **FASCICULE DE BREVET EUROPEEN**

(45) Date de publication et mention de la délivrance du brevet:
**15.08.2007 Bulletin 2007/33**

(21) Numéro de dépôt: **02700342.5**

(22) Date de dépôt: **21.01.2002**

(51) Int Cl.:
*C07K 14/00* (2006.01)　　*C07K 14/705* (2006.01)
*A61P 31/18* (2006.01)　　*C07K 17/00* (2006.01)
*A61K 38/16* (2006.01)　　*A61K 39/12* (2006.01)

(86) Numéro de dépôt international:
**PCT/FR2002/000227**

(87) Numéro de publication internationale:
**WO 2002/059146 (01.08.2002 Gazette 2002/31)**

(54) **PEPTIDES PRESENTANT UNE AFFINITE POUR LA PROTEINE VIRALE GP120, ET UTILISATION DE CES PEPTIDES**

PEPTIDE MIT AFFINITÄT ZU GP120, UND IHRE VERWENDUNGEN

PEPTIDES HAVING AFFINITY FOR THE GP120 VIRAL PROTEIN AND USE THEREOF

(84) Etats contractants désignés:
**AT BE CH CY DE DK ES FI FR GB GR IE IT LI LU MC NL PT SE TR**

(30) Priorité: **23.01.2001 FR 0100893**

(43) Date de publication de la demande:
**10.12.2003 Bulletin 2003/50**

(73) Titulaire: **COMMISSARIAT A L'ENERGIE ATOMIQUE 75015 Paris (FR)**

(72) Inventeurs:
• **VITA, Claudio**
　**F-91190 Gif sur Yvette (FR)**
• **MARTIN, Loic**
　**F-91190 Gif sur Yvette (FR)**
• **ROUMESTAND, Christian**
　**F-34990 Juvignac (FR)**
• **VEAS, Francisco**
　**F-34130 Maugio (FR)**

(74) Mandataire: **Poulin, Gérard et al Société BREVATOME 3, rue du Docteur Lancereaux 75008 Paris (FR)**

(56) Documents cités:
**US-A- 4 684 622　　US-A- 4 721 704**

• **MARTIN L ET AL: "Engineering Novel Bioactive Mini-Proteins on Natural Scaffolds" TETRAHEDRON, ELSEVIER SCIENCE PUBLISHERS, AMSTERDAM, NL, vol. 56, no. 48, 24 novembre 2000 (2000-11-24), pages 9451-9460, XP004220792 ISSN: 0040-4020**
• **VITA CLAUDIO ET AL: "RATIONAL ENGINEERING OF A MINIPROTEIN THAT REPRODUCES THE CORE OF THE CD4 SITE INTERACTING WITH HIV-1 ENVELOPE GLYCOPROTEIN." PROCEEDINGS OF THE NATIONAL ACADEMY OF SCIENCES OF THE UNITED STATES, vol. 96, no. 23, 9 novembre 1999 (1999-11-09), pages 13091-13096, XP002181795 NOV. 9, 1999 ISSN: 0027-8424 cité dans la demande**

EP 1 368 372 B1

LISTE DE SEQUENCES

<110> CEA

<120> PEPTIDE D'AFFINITE A LA PROTEINE gp 120

<130> B13685.3EE

<140>
<191>

<160> 18

<170> PatentIn Ver. 2.1

<210> 1
<211> 16
<212> PRT
<213> Homo sapiens

<220>
<223> Séquence Gln33 à Pro48 du CD4 humain

<400> 1

```
Gln Ile Lys Ile Leu Gly Asn Gln Gly Ser Phe Leu Thr Lys Gly Pro
  1               5                   10                  15
```

<210> 2
<211> 31
<212> PRT
<213> scorpion

<400> 2

```
Ala Phe Cys Asn Leu Arg Met Cys Gln Leu Ser Cys Arg Ser Leu Gly
  1               5                   10                  15

Leu Leu Gly Lys Cys Ile Gly Asp Lys Cys Glu Cys Val Lys His
              20                  25                  30
```

<210> 3
<211> 28
<212> PRT
<213> Séquence artificielle

<220>
<223> Description de la séquence artificielle: séquence issue de la scyllatoxine

<400> 3

```
Cys Asn Leu Ala Arg Cys Gln Leu Arg Cys Lys Ser Leu Gly Leu Leu
  1               5                   10                  15

Gly Lys Cys Ala Gly Ser Phe Cys Ala Cys Gly Pro
              20                  25
```

<210> 4
<211> 28
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 4

```
        Xaa Asn Leu Ala Arg Cys Gln Leu Arg Cys Lys Ser Leu Gly Leu Leu
         1               5                  10                  15


        Gly Lys Cys Ala Gly Ser Phe Cys Ala Cys Gly Pro
                    20                  25
```

<210> 5
<211> 27
<212> PRT
<213> Séquence artificielle

<220>
<223> Description de la séquence artificielle: séquence issue de la scillatoxine

<220>
<221> MOD_RES
<222> (1)
<223> acide thiopropionique

<220>
<221> MOD_RES
<222> (23)
<223> bi-phénylalanine ou naphtylalanine

<400> 5

```
        Xaa Asn Leu His Phe Cys Val Gln Arg Cys His Ser Leu Gly Leu Leu
         1               5                  10                  15


        Gly Lys Cys Ala Gly Ser Xaa Cys Ala Cys Val
                    20                  25
```

<210> 6
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 6

```
Xaa Asn Leu Ala Phe Cys Gln Leu Arg Cys Lys Ser Leu Gly Leu Leu
 1               5                  10                  15

Gly Lys Cys Ala Gly Ser Phe Cys Ala Cys Val
              20                  25
```

<210> 7
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 7

```
Xaa Asn Leu Ala Phe Cys Gln Leu Arg Cys Lys Ser Leu Gly Leu Leu
 1               5                  10                  15

Gly Lys Cys Ala Ser Ser Phe Cys Ala Cys Val
              20                  25
```

<210> 8
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 8

```
Xaa Asn Leu Ala Phe Cys Gln Leu Arg Cys Lys Ser Leu Gly Leu Leu
 1               5                  10                  15

Gly Lys Cys Ala Gly His Phe Cys Ala Cys Val
            20                  25
```

<210> 9
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 9

```
Xaa Asn Leu Ala Phe Cys Gln Leu Arg Cys Lys Ser Leu Gly Leu Leu
 1               5                  10                  15

Gly Lys Cys Ala Gly Asn Phe Cys Ala Cys Val


            20                  25
```

<210> 10
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<220>
<221> MOD_RES
<222> (23)
<223> bi-phénylalanine ou naphtylalanine

<400> 10

```
        Xaa Asn Leu Gln Phe Cys Gln Leu Arg Cys Lys Ser Leu Gly Leu Leu
         1               5                   10                      15

        Gly Lys Cys Ala Gly Ser Xaa Cys Ala Cys Val
                     20                   25
```

<210> 11
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<220>
<221> MOD_RES
<222> (23)
<223> bi-phénylalanine ou naphtylalanine

<400> 11

```
        Xaa Asn Leu His Phe Cys Gln Leu Arg Cys Lys Ser Leu Gly Leu Leu
         1               5                   10                      15

        Gly Lys Cys Gln Gly Ser Xaa Cys Thr Cys Val
                     20                   25
```

<210> 12
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<220>
<221> MOD_RES
<222> (23)
<223> bi-phénylalanine ou naphtylalanine

<400> 12

```
Xaa Asn Leu Ala Arg Cys Gln Leu Arg Cys Lys Ser Leu Gly Leu Leu
 1               5                  10                      15


Gly Lys Cys Ala Gly Ser Xaa Cys Ala Cys Val
            20                  25
```

<210> 13
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<220>
<221> MOD_RES
<222> (23)
<223> bi-phénylalanine ou naphtylalanine

<400> 13

```
Xaa Asn Leu His Phe Cys Gln Leu Arg Cys Lys Ser Leu Gly Leu Leu
 1               5                  10                      15


Gly Lys Cys Ala Gly Ser Xaa Cys Ala Cys Val
            20                  25
```

<210> 14
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<220>
<221> MOD_RES
<222> (23)
<223> bi-phénylalanine ou naphtylalanine

<220>
<221> MOD_RES
<222> (21)
<223> isomère D de Asp

<400> 14


```
Xaa Asn Leu His Phe Cys Gln Leu Arg Cys Lys Ser Leu Gly Leu Leu
 1               5                   10                  15

Gly Lys Cys Ala Xaa Ser Xaa Cys Ala Cys Ile
            20                  25
```


<210> 15
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 15


```
Xaa Asn Leu His Phe Cys Val Gln Arg Cys His Ser Leu Gly Lys Leu
 1               5                   10                  15

Gly Lys Cys Ala Gly Ser Phe Cys Ala Cys Val
            20                  25
```


<210> 16
<211> 27
<212> PRT
<213> Séquence artificielle

<220>
<223> Description de la séquence artificielle: séquence issue de la scyllatoxine

<220>
<221> MOD_RES
<222> (1)
<223> acide thiopropionique

<400> 16


```
Xaa Asn Leu His Phe Cys Val Gln Arg Cys His Ser Leu Gly Leu Lys
 1               5                   10                  15

Gly Lys Cys Ala Gly Ser Phe Cys Ala Cys Val
            20                  25
```


<210> 17

<211> 27
<212> PRT
<213> Séquence artificielle

<220>
<223> Description de la séquence artificielle: séquence issue de la scyllatoxine

<400> 17

```
Cys Asn Leu Ala Arg Cys Gln Leu Ser Cys Lys Ser Leu Gly Leu Lys
 1               5               10                      15


Gly Gly Cys Gln Gly Ser Phe Cys Thr Cys Gly
              20              25
```

<210> 18
<211> 33
<212> PRT
<213> Séquence artificielle

<220>
<223> Description de la séquence artificielle: séquence issue de la charybdotoxine

<400> 18

```
Val Ser Cys Thr Thr Ser Lys Glu Cys Trp Ser Val Cys Gln Arg Leu
 1               5               10                      15


His Asn Thr Ser Lys Gly Gly Cys Gln Gly Ser Phe Cys Thr Cys Gly
              20              25                      30


Pro
```

## Description

### Domaine technique

La présente invention se rapporte à une famille de peptides présentant une affinité et une spécificité élevées pour la protéine virale gp120, à des procédés de fabrication de ces peptides, et à l'utilisation de ces peptides.

La protéine virale gp120 est une glycoprotéine de l'enveloppe du virus de l'immunodéficience. Elle intervient dans la première étape du cycle de réplication du virus, c'est à dire dans l'étape de reconnaissance de la protéine CD4 de la membrane des lymphocytes par l'enveloppe du virus, et de fusion des membranes pour l'internalisation de la nucléocapside. Il s'agit en fait de la protéine la plus externe du site de reconnaissance de la protéine CD4 par l'enveloppe du virus.

Il existe de nombreux virus de l'immunodéficience comportant une protéine de l'enveloppe analogue à la protéine virale gp120. Parmi ceux-ci on peut citer le virus de l'immunodéficience humaine (VIH), le virus de l'immunodéficience simienne (VIS), le virus de l'immunodéficience des ovidés (VISNA), le virus de l'immunodéficience bovine (VIB), et le virus de l'immunodéficience des félins (VIF).

Certains peptides de la présente invention sont capables de se fixer à la protéine virale gp120 de l'enveloppe virale, d'inhiber la fixation de la protéine virale gp120 au récepteur CD4, avec des valeurs de $CI_{50}$ entre 0,1 et 400 nM en fonction de leur séquence. Certaines de ces molécules sont capables d'inhiber l'infection de lymphocytes T par le virus VIH avec par exemple une $DE_{50}$ (Dose Effective 50%) de 100 à 900 nM. En outre, la fixation de ces molécules à la protéine virale gp120 recombinante induit une variation conformationnelle dans celle-ci qui démasque de nouveaux épitopes, avec la même efficacité que la molécule CD4 soluble.

Les peptides de la présente invention peuvent trouver des applications thérapeutiques et vaccinales. Ils permettent aussi la conception de nouvelles molécules utiles, par exemple, dans la détection mais aussi dans la purification de la protéine de l'enveloppe du VIH et dans la découverte de nouveaux médicaments anti-SIDA.

### Art antérieur

De nombreuses recherches sont menées pour trouver des traitements et des moyens de prévention efficaces de l'infection par le virus de l'immunodéficience et notamment de l'immunodéficience humaine (VIH). Les difficultés majeures concernent le développement de vaccins et de traitements immunothérapeutiques efficaces. Ceci est dû en particulier à la variabilité du virus, à la difficulté de compréhension du mécanisme de son entrée dans les cellules cibles et de la réponse immunitaire nécessaire pour la protection de l'infection par le virus.

Dans la description qui suit, les références entre crochets renvoient aux documents de la liste de références annexée.

Comme le démontre l'analyse de la structure tridimensionnelle du complexe CD4-gp120 [1] (voir références annexées), les éléments structuraux du CD4 critiques pour sa liaison à la glycoprotéine virale gp120 incluent les acides aminés Gly38, Gln40, Gly41, Ser42, Phe43, Thr45, qui sont compris dans la région 36-47, et l'arginine-59 du CD4. La région 36-47 du CD4 a été assimilée à la région CDR2 des immunoglobulines et présente une structure ordonnée en épingle à cheveux. Cette structure est formée d'un brin β (C'), correspondant à la séquence 36-40, suivie d'un coude correspondant à la séquence 40-43 qui permet à un deuxième brin β (C"), correspondant à la séquence 43-47, de former avec le premier une structure β antiparallèle. Cette structure est stabilisée par des liaisons hydrogène entre l'azote amidique des résidus Gly38, Gln40, Phe43, Thr45, Gly47 du premier brin β et l'oxygène carbonylique des résidus, respectivement, Thr45, Phe43, Gln40, Gly38, Ile36 du deuxième brin β. Cette structure en épingle à cheveux a un rôle central dans l'interaction du CD4 avec la protéine virale gp120 et accomplit une double fonction: premièrement, elle permet aux acides aminés Gly38, Gln40, Gly41, Ser42, Phe43 et Thr45 de former une surface moléculaire complémentaire à la surface d'interaction de la protéine virale gp120 et, en particulier, permet à la chaîne latérale de la Phe43 de s'insérer à l'entrée d'une cavité hydrophobe dans la surface moléculaire de la protéine virale gp120 ; deuxièmement, elle permet une interaction de type feuillet β entre les squelettes polypeptidiques du brin β C" du CD4 et le brin β15 (résidus 365-368) de la protéine virale gp120. L'arginine 59 (Arg59) a aussi un rôle critique dans l'interaction CD4-gp120, puisque le groupement guanidinium de sa chaîne latérale forme un double pont hydrogène avec la chaîne latérale du résidu Asp368 de la protéine virale gp120, permettant la stabilisation de l'interaction de type structure β entre les brins C" du $CD_4$ et β15 de la protéine virale gp120. Des expériences de mutagenèse dirigée ont démontré que tous ces résidus du CD4 jouent un rôle critique dans l'interaction avec la protéine virale gp120 [2], [3].

La reproduction de cette structure ordonnée en épingle à cheveux et de la conformation des chaînes latérales de ses résidus est critique dans une molécule qui doit se fixer sur la protéine virale gp120 au site d'interaction du CD4. Le fait que des constructions peptidiques simples, comme un peptide linéaire correspondant à la séquence 37-53 et un peptide cyclique correspondant à la séquence 37-46 du CD4, ne possèdent pas une affinité mesurable pour la protéine virale gp120, en est une démonstration [4].

**EP 1 368 372 B1**

L'infection des cellules CD4 par le VIH est médiée par une interaction de haute affinité entre l'enveloppe virale et le CD4. Des expériences de mutagénèses et de compétition avec des anticorps ont permis de localiser dans le domaine D1 du CD4, la surface de contact avec la protéine gp120 de l'enveloppe virale. La structure tridimensionnelle d'une forme recombinante fonctionnelle de la glycoprotéine gp120, mais délétée des boucles V1-V2-V3 et des régions N- et C-terminales, en complexe avec les domaines D1D2 du CD4 et avec le fragment Fab d'un anticorps monoclonal, a été résolue récemment par cristallographie [1]. Dans cette structure, le CD4 interagit avec une large dépression de la surface moléculaire (800 Å$^2$) de la protéine virale gp120, en utilisant une large surface moléculaire (742 Å$^2$), qui est centrée autour de la région qui a été assimilée à la région CDR2 des immunoglobulines. Au coeur de la dépression moléculaire de la protéine virale gp120 s'ouvre une cavité étroite et profonde, la "cavité Phe43", dans laquelle la chaîne latérale de la Phe43, au sommet de la région CDR2 du CD4, occupe l'entrée. La résolution de cette structure confirme les données précédentes de mutagénèse, qui indiquent le résidu Phe43 et toute la boucle CDR2 comme fonctionnellement très importants, et propose cette dernière comme cible possible pour l'inhibition de l'attachement des virions VIH-1 aux cellules. L'interaction gp120-CD4 permet la fixation du virus à la membrane des cellules cibles et représente la première interaction protéine-protéine dans le mécanisme de l'infection. Néanmoins, d'autres co-récepteurs sont nécessaires pour une entrée efficace du VIH dans les cellules. Il s'agit du CXCR-4 [5], [6], qui est impliqué dans l'entrée des souches virales lymphotropiques (X4) et du CCR-5 [7], [8], qui est impliqué dans l'entrée des souches M-tropiques (R5) et de la plupart des isolats primaires. Plusieurs preuves indiquent que la fixation de la protéine virale gp120 sur le CD$_4$ produirait des variations conformationnelles dans la glycoprotéine de l'enveloppe, qui exposeraient de nouveaux sites, détectables par des anticorps spécifiques, dénommés CD4i, et augmenteraient l'affinité de l'enveloppe pour les récepteurs des chemokines [9], [10], les co-récepteurs de l'entrée. Après fixation du CD4 et du co-récepteur par la protéine virale gp120, d'autres variations conformationnelles conduiraient à une réorganisation structurale de la gp41, qui aboutirait à l'exposition de son peptide fusogène, puis à la fusion des membranes virales et cellulaires et enfin à l'entrée de la charge virale dans la cellule.

La structure cristallographique de la protéine virale gp120 complexée au CD4 a permis d'élucider un des mécanismes qu'utilise le VIH pour échapper à la réponse immune. On savait déjà que la protéine de l'enveloppe gp120 possédait des régions hypervariables et fortement glycosylées. La structure cristallographique a démontré en fait que les régions hypervariables et les régions hautement glycosylées forment la surface moléculaire exposée de la protéine virale gp120, qui correspond aussi à la surface accessible des virions. Seules quelques régions de la surface moléculaire de la protéine virale gp120 sont conservées et peuvent être la cible des anticorps. Ces régions ne sont pas normalement accessibles et sont probablement protégées par les boucles hypervariables (V1-V2-V3) qui dans la protéine cristallisée ont été délétées, et sont donc invisibles. Une de ces régions est une surface proche du site d'interaction pour les récepteurs des chemokines, et accessibles aux anticorps CD4i, après fixation de l'enveloppe au CD4. Le site de liaison au CD4 est une autre surface exposée et conservée : cependant, cette surface est présente dans une cavité de la protéine virale gp120 et n'est donc pas accessible aux anticorps, mais peut accommoder le CD$_4$ qui présente un seul domaine d'immunoglobuline à la différence des anticorps qui utilisent deux domaines pour la reconnaissance moléculaire.

Les outils aujourd'hui disponibles pour la reconnaissance de la protéine de l'enveloppe (gp120) sont limités. Il s'agit des anticorps CD4i [11], [12], [13], qui sont capables de fixer un large spectre d'enveloppes primaires ; toutefois, leur affinité pour l'enveloppe virale est faible et augmente seulement en présence de CD4 ; ce qui ne rend pas leur utilisation facile et aisée. D'autres anticorps, tel le IgG1b12, F105 ou 15e ne seraient pas capables de reconnaître tous les isolats primaires. Une molécule de CD$_4$, marquée avec la biotine pourrait aussi représenter une solution. Cette molécule est commercialement disponible (Intracel). Malheureusement, son prix est élevé et ses propriétés fonctionnelles de fixation de la protéine virale gp120 ont été fortement diminuées (de 100 fois) par la réaction de biotinylation, ce qui limite fortement son utilisation.

L'inhibition de l'interaction de la protéine virale gp120 avec le récepteur principal de l'entrée, le CD4, par un CD4 recombinant soluble et/ou par des chimères d'immunoglobulines contenant des domaines du CD4, a été l'une des premières approches thérapeutiques suggérées et expérimentées pour l'inhibition de l'infection par VIH-1. Ces molécules sont efficaces in vitro dans l'inhibition de l'infection virale seulement dans le cas de souches adaptées au laboratoire, mais sont inefficaces dans le cas de nombreux isolats primaires. La dégradation rapide in vivo de ces constructions dérivées du CD4 et leur affinité plus faible pour l'enveloppe virale des isolats primaires ont été proposées comme causes principales de leur inefficacité. Cependant, des études ont démontré que l'affinité, pour le CD4, des gp120 recombinantes monomériques de certains isolats primaires n'est pas significativement différente de celle de gp120 issues de souches de laboratoire. La nature oligomérique de la protéine virale gp120 à la surface du virus, la densité et la structure du CD4 à la surface des cellules permissives et peut être la présente d'autres interactions moléculaires non encore clairement élucidées ou d'autres molécules accessoires d'entrée pourraient jouer un rôle important dans le mécanisme de l'entrée et représenter d'autres causes de l'inefficacité des protéines CD4 solubles comme antagonistes de l'entrée. Le développement d'agents antiviraux qui ciblent la protéine virale gp120 présente un challenge majeur, à cause de la grande variabilité génétique de la protéine de l'enveloppe, facteur qui peut expliquer l'inefficacité de beaucoup d'inhibiteurs de la protéine virale gp120. Néanmoins, le fait que les résidus qui contribuent à former le coeur du site de liaison de la protéine virale gp120 pour le CD4 sont formés par des résidus très conservés suggère que des inhibiteurs de la protéine virale gp120 peuvent être efficaces contre un large spectre d'isolats VIH-1.

Des constructions peptidiques dérivées du CD4 ont été proposées comme inhibiteurs de l'infection virale : il s'agit d'un peptide mimétique structural de la boucle CDR2 du CD4 qui incorpore un groupement phényl mimant la Phe43 [14] et un peptide cyclique correspondant à la boucle CDR3 du CD4 et incorporant des résidus aromatiques additionnels [15]. Cependant, ces constructions présentent une activité antivirale faible limitée à un isolat de laboratoire et, même si elles ont été conçues au départ comme mimétiques structuraux du CD4, elles ne sont pas actives dans l'étape d'entrée [16].

Actuellement, un certain nombre d'inhibiteurs de l'interaction gp120-CD4 ont été proposés et présentent une application clinique. Il s'agit par exemple d'une protéine recombinante de grosse taille, formée par la fusion génétique des domaines DID2 du CD4 avec les domaines constants d'une immunoglobuline IgG2 [17]. Cette construction PR0542 est en phase clinique I/II. Cette large protéine recombinante semble être bien tolérée par l'organisme humain, mais son efficacité dépend d'une dose circulante élevée difficile à atteindre. Des molécules de plus petites tailles ont été proposées comme inhibiteurs de l'interaction CD4-gp120 et sont en phase de test clinique. Il s'agit : d'un colorant bis-azo FP21399 [18], identifié par une approche combinatoire, du phosphorothioate oligonucléotide zintevir (AR177) [19], d'un polymère à base de naphtalène sulphonate, PR02000 [20] et d'un dérivé d'amidon, le dextrin 2-sulfate (D2S) [21], qui inhibent des isolats chimiques VIH-1 dans des cultures cellulaires, mais exigent des concentrations élevées. Parmi les inhibiteurs de l'entrée, il y a aussi le SPC3 [22], une construction peptidique synthétique multibranchée, qui, proposée à l'origine comme un inhibiteur de l'interaction CD4-gp120, est ensuite révélée active dans une étape suivant l'attachement des virions aux cellules. D'autres molécules de petite taille ont été proposées comme inhibiteurs de l'entrée : le bicyclam AMD3100 [23], le NSC651016 [24], le peptide T22 [25] et sa version plus récente T134 ou T140 [26] et le TAK779 [27]. Ces molécules sont des inhibiteurs de l'entrée virale, actifs sur les co-récepteurs de l'entrée CXCR4 et CCR5, mais n'ont aucune activité dans l'interaction CD4-gp120. Des peptides dérivés de la séquence de la région C-terminale de la glycoprotéine gp41, le peptide T20 (ou DP178) [28] et sa plus récente version améliorée DT1249 [29], sont d'autres inhibiteurs de l'infection qui ciblent une phase transitoire de l'entrée, ultérieure à l'attachement de l'enveloppe virale au CD4 et précédant la fusion des membranes virus cellule : ces peptides sont inactifs dans l'interaction gp120-CD4. La plupart de ces produits sont actuellement en test clinique de phase I/II, mais leur efficacité thérapeutique reste encore à vérifier.

Le régime thérapeutique anti-SIDA actuellement utilisé en clinique, la trithérapie, comporte une combinaison de trois inhibiteurs qui ciblent deux enzymes virales, la transcriptase inverse et la protéase. Même si la trithérapie est arrivée à réduire significativement la charge virale de beaucoup de malades, elle n'est pas capable même dans les cas les plus favorables d'éradiquer la maladie, car des réservoirs dormants d'infection persistent toujours [30]. Le succès partiel de la trithérapie d'une part et l'impossibilité de stopper l'infection SIDA avec les protocoles thérapeutiques actuels d'autre part, ont augmenté la demande de nouveaux médicaments pouvant être actifs au niveau d'autres cibles virales et augmenter le répertoire et l'efficacité des médicaments cliniques actuels.

Des expériences dans le laboratoire de J.H. Nunberg, Montana Biotechnology Center, The University of Montana, Missoula, USA, ont démontré que le pontage chimique de formes protéiques présentes dans l'étape précédent la fusion virus-cellule représentent de nouveaux immunogènes qui sont capables d'induire la formation d'anticorps neutralisant le virus [31]. Le complexe de pré-fusion de l'enveloppe virale avec les récepteurs de l'entrée représente donc une structure de grand intérêt actuellement, parce que cette structure pourrait représenter la composante la plus importante d'une formation protéique dans la préparation d'un vaccin contre le SIDA. Des expériences dans le laboratoire de A. DeVico, Institute of Human Virology, University of Maryland, Baltimore, USA, ont démontré que le complexe gp120-CD4, stabilisé par pontage chimique ou comme protéine de fusion recombinante, représente une forme macromoléculaire qui expose des sites antigéniques cryptiques de la protéine virale gp120 et qui est capable d'induire des anticorps neutralisants [32], [33]. L'obtention de ce complexe antigénique par pontage chimique n'est pas satisfaisante car non homogène et difficile à reproduire. Son obtention comme protéine recombinante ne conduit pas à une forme structuralement stable et efficace. Mais surtout, la présence du CD4 dans ces préparations pourrait présenter le danger d'induire une réponse auto-immune dans un organisme déjà immunologiquement déprimé.

Par ailleurs, la production de l'enveloppe virale dans sa forme recombinante monomérique (gp120) ou dans la forme plus native trimérique (gp140 ou analogues) présente un intérêt très important dans la recherche et dans la production à large échelle de formes recombinantes, potentiellement utilisables pour la vaccination. La purification de ces formes recombinantes n'est pas aisée et implique l'utilisation de supports chromatographiques fonctionnalisés avec des ligands, surtout des lectines, qui ne sont pas très spécifiques pour la protéine de l'enveloppe. L'utilisation d'un support chromatographique, contenant le CD4 recombinant fixé covalement, introduirait une étape plus spécifique et efficace pour la purification de la protéine virale gp120, mais le coût d'un tel support serait très élevé et cette protéine ne serait pas suffisamment stable dans toutes les conditions d'utilisation.

Le document MARTIN et al., « Engineering Novel Bioactive Mini-Proteins on Natural Scaffolds » TETRAHEDRON, vol. 56, 24 novembre 2000, pages 9451-9460 décrit des peptides CD4 mais ceux-ci ne comportent pas un acide thiopropionique (TPA) à l'extrémité -C terminale conformément aux peptides de la présente invention. Ils présentent des concentrations d'inhibition (C150%) beaucoup plus élevées que celles de la présente invention.

Le document US-A-4 721 704 (CHANG JAW-KANG) décrit des peptides auriculaires qui ont une activité natriurétique, diurétique et antihypertensive. Ces peptides ont 21 ou 22 acides amines et sont de séquence :

Mpr-Phe-DAla-Gly-Arg-Ile-Asp-Arg-Ile-Gly-Ala-Gln-
-Ser-$R_1$-Leu-$R_2$-Cys-Asn-Ser-Phe-Arg-$R_3$
15                                        20

où Mpr est un dérivé de l'acide $\beta$-mercaptopropanoique qui remplace un Cys ($R_1$ et $R_2$ étant Gly ou DAla, et $R_3$ étant OH, $NH_2$, Tyr-OH ou Tyr-$NH_2$). Outre le fait que les peptides décrits sont sans rapport avec ceux de la présente invention, ce document ne décrit pas d'activité biologique associée à l'acide $\beta$-mercaptopropanoique.

Le document US-A-4 684 622 (HUFFMAN WILLIAM F et al.) décrit des peptides qui sont des dérivés de la vasopressine et ont une séquence:

$CH_2CO$-X-P-Y-Asn-Cys-Z-A
|
C-$(R)_2$
|
S ——————————— S

Un acide mercaptopropionique peut être présent en position 1. Outre le fait que les peptides décrits dans ce document sont sans rapport avec ceux de la présente invention, aucune activité biologique n'est associée à l'acide mercaptopropionique.

Les documents H. OZAKI et al.: « Molecular Structure of the Toxic Domain of Heat-Stable Enterotoxin Produced by a Pathogenic Strain of Escherichia coli. », THE JOURNAL OF BIOLOGICAL CHEMISTRY, Vol. 266, 25 mars 1991, pages 5934-5941 et EP 0 989 136 ne décrivent pas de peptides CD4. Ils décrivent respectivement une entérotoxine produite par E. Coli, et un peptide cyclique ayant un effet sur la restauration de mutants de la protéine P53. Rien dans ces documents ne laisse penser qu'un TPA en position 1 peut modifier l'activité biologique des peptides qui y sont décrits.

Exposé de l'invention

La présente invention fournit une famille de peptides qui miment le CD4 et pallient les inconvénients précités de l'art antérieur. En effet elle fournit un peptide stable, présentant une très forte affinité pour l'enveloppe du virus du SIDA, en particulier pour la protéine gp120.

Le peptide de la présente invention est caractérisé en ce qu'il comprend la séquence (I) suivante :

```
TPA- Xaaᵃ- Xaaᵇ- Ala ou Gln ou His - Arg ou Phe -
    1                                              5

Cys - Xaaᶜ- Xaaᵈ- Arg - Cys - Lys - Xaaᵉ- Xaaᶠ-
                             10

Xaaᵍ- Xaaʰ- Leu ou Lys - Xaaⁱ - Lys - Cys - Ala
              15

ou Gln -Gly ou (D)Asp ou Ser - Ser ou His ou Asn -
    20

Xaaʲ- Cys - Thr ou Ala -Cys - Xaaᵏ-NH₂,
              25
```

dans laquelle TPA représente l'acide thiopropionique, Xaaᵃ, Xaaᵇ, Xaaᶜ, Xaaᵈ, Xaaᵉ, Xaaᶠ, Xaaᵍ, Xaaʰ, et Xaaⁱ, sont des acides aminés, naturels ou non naturels, identiques ou différents, Xaaʲ représente Nal, Phe ou Bip, et Xaaᵏ représente Gly, Val ou Ile.

Le résidu (D)Asp représente l'isomère optique D de l'acide aspartique, Nal représente la β-naphtylalanine, et Bip représente la bi-phénylalanine.

Les inventeurs ont mis en évidence que le peptide de la présente invention défini par la séquence (I), comportant un acide thiopropionique à la position 1 de la séquence, un résidu Phe, Nal, ou Bip en position 23 de la séquence (Xaaʲ), et un résidu Gly, Val ou I le en position 27 de la séquence (Xaaᵏ) présente une forte affinité et une grande spécificité de liaison pour la protéine gp120 de l'enveloppe virale du virus de l'immunodéficience.

Ce peptide comporte 27 ou 28 résidus seulement, et présente une structure en épingle à cheveux constituée de deux brins β antiparallèles reliés par un coude β de quatre résidus. Les deux brins antiparallèles, étant à une distance pouvant donner des interactions à ponts hydrogène intramoléculaires, stabilisent la structure. Notamment, la distance entre les atomes azote amidique et oxygène carbonylique de la liaison peptidique est de 2,5 à 3,6 Å. Cette structure bien définie et stable reproduit des éléments structuraux du CD4 critiques pour sa liaison à la glycoprotéine gp120.

La structure tridimensionnelle du peptide de la présente invention a été résolue expérimentalement par résonance magnétique nucléaire (RMN). Cette analyse démontre que l'organisation tridimensionnelle de ce peptide reproduit le squelette peptidique de la structure en épingle à cheveux du CD4, et la région 37-46 de ce peptide peut être superposée à la région 36-37 du CD4 avec une déviation rms de seulement 1,05 Å. En outre, les chaînes latérales des résidus Ala ou Gln 20, Ser 22, Xaaʲ 23 (Nal, Phe ou Bip), et Thr ou Ala 25 ont une orientation comparable à celle des résidus correspondants du CD4, à savoir Gln 40, Ser 42, Phe 43, et Thr 45. En particulier, la chaîne latérale de Xaaʲ 23 pointe du motif en épingle à cheveux dans une conformation semblable à celle de la Phe 43 du CD4 pour s'insérer à l'entrée d'une cavité hydrophobe de la protéine virale gp120, renforçant ainsi le lien avec gp120. Arg et Lys aux positions 9 et 18 sont topologiquement équivalents aux résidus Arg59 et Lys35 de la protéine CD4.

TPA et les 5 cys du peptide forment des ponts disulfures qui présentent un appariement de type 1-3, 2-4 et 3-6 et stabilisent la structure en épingle à cheveux.

Le motif structural en épingle à cheveux a une surface accessible au solvant et/ ou à une molécule de plus grosse taille telle qu'une protéine, ce qui peut favoriser son interaction avec la protéine de l'enveloppe virale.

Les chaînes latérales des acides aminés de la surface accessible au solvant de ce motif sont proches dans l'espace et forment une surface moléculaire continue qui reproduit la structure de plusieurs résidus importants pour la liaison CD4-gp120.

La plate-forme structurale qui contient le motif structural en épingle à cheveux contient aussi d'autres régions structurales capables d'accueillir des fonctionnalités chimiques additionnelles dans une position spatiale bien définie par rapport au motif structural en épingle à cheveux pouvant ainsi renforcer sa fonction biologique de liaison et/ou présenter des groupement de marquage. Par exemple, un groupement de biotine ou un groupement de fluorescéine a été incorporé au niveau de la chaîne latérale de la lysine 11, sans provoquer de perte de l'activité biologique du dérivé. L'incorporation de ces groupements a permis l'utilisation de ces dérivés dans des tests d'interaction avec la protéine de l'enveloppe, comme décrit ci-dessous.

Selon l'invention, la séquence (I) peut comprendre en outre un résidu proline lié au résidu Xaaᵏ. Un résidu proline ajouté en position 28 stabilise l'extrémité C-terminale et rend moins flexible le brin β-terminal du motif structural en épingle à cheveux.

Selon l'invention, dans la séquence (I), xaa$^i$ peut être Gly.

Par exemple, le peptide de la présente invention peut comprendre une séquence choisie parmi les séquences ID n°4, ID n°5, ID n°6, ID n°7, ID n°8, ID n°9, ID n° 10, ID n°11, ID n°12, ID n°13, ID n°14, ID n°15 et ID n°16 de la liste de séquence annexée.

Le peptide de la présente invention peut être obtenu par synthèse chimique en phase solide ou par recombinaison génétique. Aussi, la présente invention se rapporte également à un procédé de fabrication du peptide selon l'invention tel qu'il est défini ci-dessus, ledit procédé comprenant une synthèse chimique en phase solide dudit peptide. La synthèse chimique peut être réalisée par exemple avec un synthétiseur automatique de peptide du type Applied Biosystems, mod.433A. Elle peut être réalisée par exemple en chimie Fmoc qui utilise le groupement fluoremylméthyloxycarbonyle pour la protection temporaire de la fonction α-aminique des acides aminés.

Le peptide de l'invention peut aussi être fabriqué au moyen d'un procédé comprenant les étapes suivantes :

a) intégration d'une séquence d'acide nucléique dans un vecteur d'expression, ladite séquence d'acide nucléique codant pour le peptide de la présente invention,
b) introduction du vecteur d'expression comprenant ladite séquence d'acide nucléique dans une cellule hôte,
c) culture de la cellule hôte comprenant ledit acide nucléique dans des conditions de culture permettant la synthèse dudit peptide,
d) récupération du peptide synthétisé, et
e) greffage du TPA en position N-terminale.

Les éléments techniques pour la réalisation de ce procédé de synthèse peptidique sont connus de l'homme du métier. Ils sont décrits par exemple dans l'ouvrage de Sombrook, Fritsch et Maniatis, MOLECULAR CLONING, A LABORATORY MANUAL, 2nd edit*ion.* Le greffage d'un TPA en position N-terminale du peptide peut être réalisé au moyen d'un procédé classique de chimie organique applicable à un peptide.

Les inventeurs ont synthétisé une famille de peptides reproduisant une partie de la structure de la région du CD4 ressemblant à la région CDR2 des immunoglobulines, qui, comme l'ont démontré des résultats mutagenèse et d'analyse cristallographique, sont parmi les régions critiques de la liaison CD4 à la protéine virale gp120. Les peptides de la présente invention sont capables de se fixer sur la région de la glycoprotéine gp120 qui interagit avec le CD4, le récepteur principal de l'entrée du VIH-1 dans les cellules cibles CD4.

Ils constituent une famille d'inhibiteurs dont l'affinité pour la glycoprotéine virale gp120 recombinante monomérique, varie entre des valeurs de CI$_{50}$ allant de 0,1 nM à 400 nM. Ils représentent des inhibiteurs spécifiques et puissants de l'interaction CD4-gp120 basés sur la structure du CD4. La liaison avec la protéine virale gp120 recombinante se fait en compétition avec le CD4 soluble et est spécifique de la forme bien structurée.

Grâce au mimétisme structurel et fonctionnel du CD4, ces peptides sont utilisables dans les différents domaines décrits ci-dessous.

Les peptides de la présente invention peuvent fixer l'enveloppe VIH-1 sous sa forme recombinante comme le démontrent les tests ELISA. Ces peptides, après marquage avec une sonde fluorescente ou radioactive appropriée, permettent de détecter la présence de l'enveloppe VIH-1 en solution ou à la surface d'une cellule, et donc de déceler la présence d'une cellule infectée et donc de l'infection VIH-1.

Les peptides de la présente invention inhibent l'interaction entre les protéines recombinantes CD4-gp120 in vitro et par ce biais, ils sont également capables de stopper l'entrée du virus VIH-1$_{LAI}$ (isolat primaire) et VIH-1$_{LEN}$ (isolat clinique) dans les lymphocytes circulants. Les peptides les plus puissants de la présente invention sont, en fait, capables d'inhiber l'infection des lymphocytes avec une DE$_{50}$ entre 100 et 900 nM. Ces molécules représentent donc des agents antiviraux, qui trouvent une application clinique. Du fait de leur nature peptidique, ils peuvent être utilisés par injection ou perfusion ou dans des applications locales externes.

La capacité des peptides de la présente invention d'inhiber la liaison de la protéine virale gp120 au CD4 est une démonstration de la capacité de ces peptides d'inhiber l'infection virale, car ils empêchent une des premières étapes de l'entrée virale. Ces inhibiteurs de l'entrée sont donc des composants à action antivirale. Les peptides de la présente invention représentent, donc, des molécules utilisables dans une thérapie anti-VIH.

Les peptides de la présente invention sont capables d'induire des variations de la conformation de la protéine virale gp120 recombinante et de l'enveloppe qui permettent d'être détectées par des anticorps monoclonaux spécifiques dénommés CD4i, comme le CG10, 17b et 48D. Ces variations de la conformation sont donc apparemment similaires à celles qui sont induites par la liaison du CD4. La liaison des peptides de l'invention à la protéine virale gp120 recombinante et à l'enveloppe virale démasque donc des épitopes de l'enveloppe, qui ne sont pas accessibles normalement sans le CD4. Ces épitopes représentent de nouveaux sites antigéniques, potentiellement très importants pour développer des anticorps neutralisant le VIH-1. Les peptides de la présente invention sont donc aussi utilisables dans une application vaccinale, notamment dans une formulation qui comporte son complexe avec une protéine de l'enveloppe virale.

Certains peptides de la présente invention, présentant une affinité plus faible pour la protéine virale gp120 recombinante que d'autres. Ceux-ci ont également une utilisation pratique très importante dans la purification de formes fonctionnelles de l'enveloppe virale. En effet, la fixation de la protéine virale sur ces peptides est plus facilement réversible. Pour démontrer cela, les inventeurs ont fixé covalement un de ces peptides à un support polymérique hydrophile (Sepharose 4B) utilisé en chromatographie liquide, en utilisant un bras flexible fixé du côté opposé à la surface active de la molécule. Cette molécule, ainsi fixée, peut interagir encore avec la protéine virale gp120 recombinante et est capable de retenir cette protéine virale sur la matrice polymérique. Elle peut ensuite être détachée par une acidification du milieu. Un support ainsi fonctionnalisé permet donc de purifier la protéine virale gp120 recombinante, et éventuellement d'autres formes de l'enveloppe virale et même le virus entier, dans des préparations à large échelle.

En outre, les peptides de la présente invention peuvent être marqués par exemple avec de la fluorescéine et de la biotine ou radio-marqués, sans que l'affinité de liaison à la protéine de l'enveloppe virale gp120 soit altérée. Ces peptides marquées peuvent être utilisés en tant que traceurs, dans des expériences de compétition de leur liaison à la gp120 par exemple pour effectuer un criblage moléculaire. Les méthodes utilisables sont celles qui sont accessibles à l'homme du métier dans la littérature qui se rapporte à l'étude des interactions moléculaires (voir par exemple [37, 38, 39, et 40]. Dans un procédé de criblage, toute molécule capable d'inhiber l'interaction entre un peptide de la présente invention et une protéine virale gp120 peut représenter un inhibiteur de l'interaction CD4-gp120, et donc un inhibiteur de l'infection virale. Il est donc possible, grâce aux peptides de la présente invention de cribler toute molécule capable d'interagir avec la protéine gp120 ou une protéine analogue à la gp120 en particulier, des molécules à activité antivirale ou des molécules utiles pour la fabrication de médicament.

La présente invention se rapporte donc également à l'utilisation du peptide selon l'invention tel qu'il est défini ci-dessus, pour préparer un médicament.

Elle se rapporte également à l'utilisation du peptide selon l'invention tel qu'il est défini ci-dessus, pour préparer un agent antiviral.

Elle se rapporte également à l'utilisation du peptide selon l'invention tel qu'il est défini ci-dessus, pour préparer un médicament destiné au traitement du SIDA.

Elle se rapporte également à un vaccin comprenant un complexe d'un peptide selon l'invention et une protéine d'enveloppe d'un virus. Le virus peut être un virus du SIDA tel que ceux précités.

La présente invention se rapporte également à l'utilisation du peptide selon l'invention tel qu'il est défini ci-dessus, pour fabriquer un produit de diagnostic, par exemple du SIDA.

Elle se rapporte également à l'utilisation d'un peptide selon l'invention tel qu'il est défini ci-dessus pour la détection de molécules de l'enveloppe virale du VIH.

Elle se rapporte également à l'utilisation du peptide selon l'invention tel qu'il est défini ci-dessus pour un criblage de molécules inhibitrices de l'interaction de la gp120 ou de ses analogues avec la molécule CD4, pour le criblage de molécules à activité antivirale et pour le criblage de molécules utiles pour la fabrication de médicament.

Elle se rapporte également à l'utilisation du peptide selon l'invention tel qu'il est défini ci-dessus, comme ligand immobilisé pour fonctionnaliser une matrice de chromatographie.

D'autres avantages encore apparaîtront à la lecture des exemples suivants en référence à la liste de séquences et aux figures annexées.

**Brève description de la liste des séquences**

La liste des séquences annexée fournit les séquences peptidiques suivantes :

- séquence ID n°1 : séquence sCD4.
- séquence ID n°2 : séquence de la scyllatoxine (ScTx).
- séquence ID n°3 : séquence CD4M9 : peptide issu de la scyllatoxine, ne présentant pas de TPA en position 1 de la séquence.
- séquences ID n°4-16 : séquences nommées CD4M9T, CD4M26, CD4M27, CD4M27A, CD4M27B, CD4M27C, CD4M30, CD4M31, CD4M32, CD4M33, CD4M35, K15 et K16 respectivement de la présente invention.
- séquence ID n°17 : séquence nommée CD4M3 issue de la scyllatoxine ne présentant pas de TPA en position 1 de la séquence.
- séquence ID n°18 : séquence nommée CD4MO issue de la charybdotoxine.

**Brève description des figures**

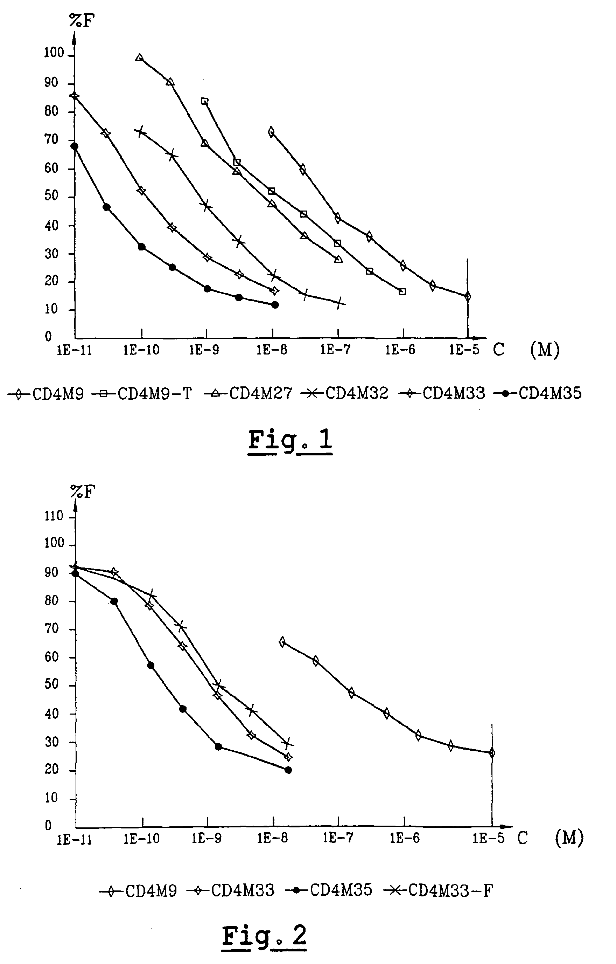

- Figure 1 : Courbes d'inhibition de la liaison du CD4 à la protéine virale gp120, par des peptides de la présente invention, obtenues par ELISA en compétition. Les peptides testés sont: CD4M9, CD4M9T, CD4M27, CD4M32, CD4M33 et CD4M35. Les données expérimentales sont reportées en pourcentage de liaison (%F) en fonction de la concentration des peptides.

- Figure 2 : Courbe d'inhibition de la liaison du CD4 à la protéine virale gp120, par le peptide marqué CD4M33-fluorescéine, CD4M33-F, obtenue par ELISA en compétition. Les courbes des peptides CD4M9 (séquence ID n°3), CD4M33 (séquence ID n°13) et CD4M35 (séquence ID n°14) sont aussi représentées pour comparaison. Les données expérimentales sont reportées en pourcentage de liaison (%F) en fonction de la concentration des peptides.

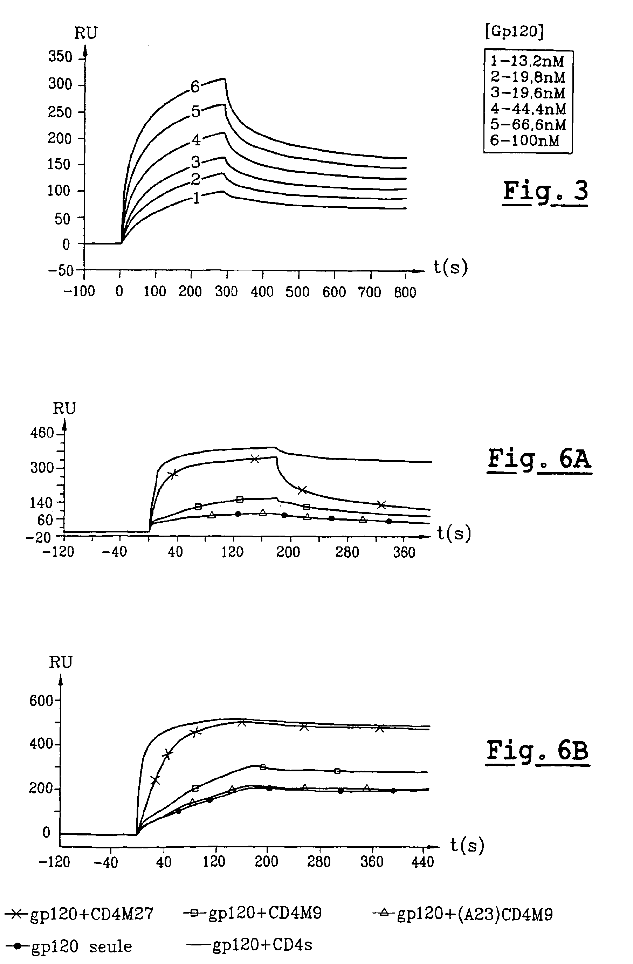

- Figure 3 : Courbes d'interaction de la protéine virale recombinante gp120-HXB2 avec le peptide CD4M33 (séquence ID n°13) fixé à la surface d'une puce d'un instrument de résonance plasmonique de surface, Les courbes d'association (0-300 s) et de dissociation (300-800 s) ont été enregistrées après injection de la protéine virale gp120 aux concentrations 13,2(1), 19,8 (2), 29,6 (3), 44,4 (4) , 66,6(5) et 100(6) nM. Les données sont reportées en unités de résonance (RU) en fonction du temps (t) en seconde.

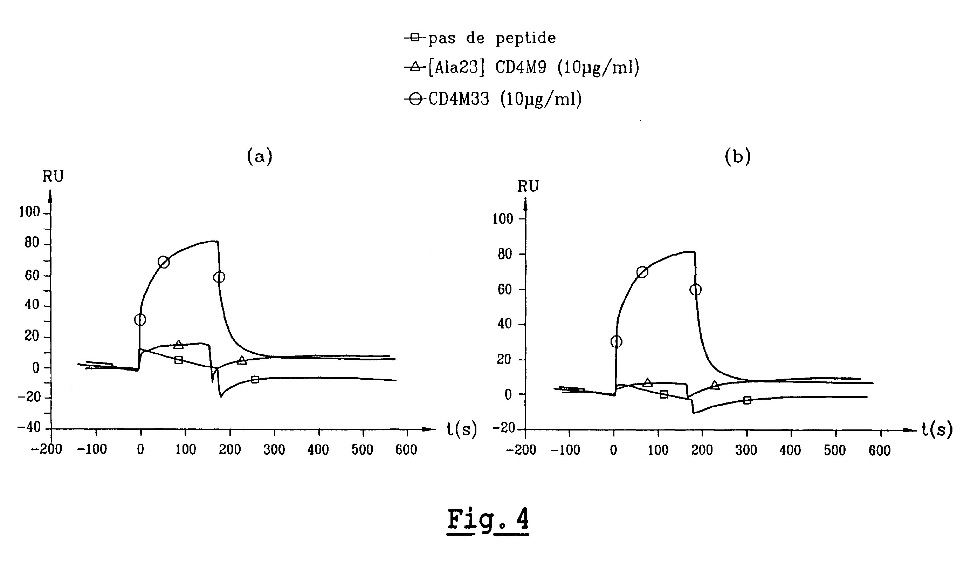

- Figure 4(a) et (b) : courbe d'interaction du VIH-1, inactivé par AT-2 [34], avec l'anticorps 48d [11] (a) et CG10 [35], (b), fixés à la surface d'une puce d'un instrument de résonance plasmonique de surface en présence du peptide CD4M33 (10 μg/ml), d'un peptide inactif (Ala23) CD4M9 (Phe23 remplacé par Ala dans la séquence peptidique de CD4M9) (10 μg/ml) et en absence de peptide. Les courbes d'association (0-180 s) et de dissociation (180-600 s) ont été enregistrées après injection du VIH-1, qui a été pré-incubé avec les peptides à 20°C pendant 1h. Les données sont reportées en unité de résonance (RU) en fonction du temps (t) en seconde.

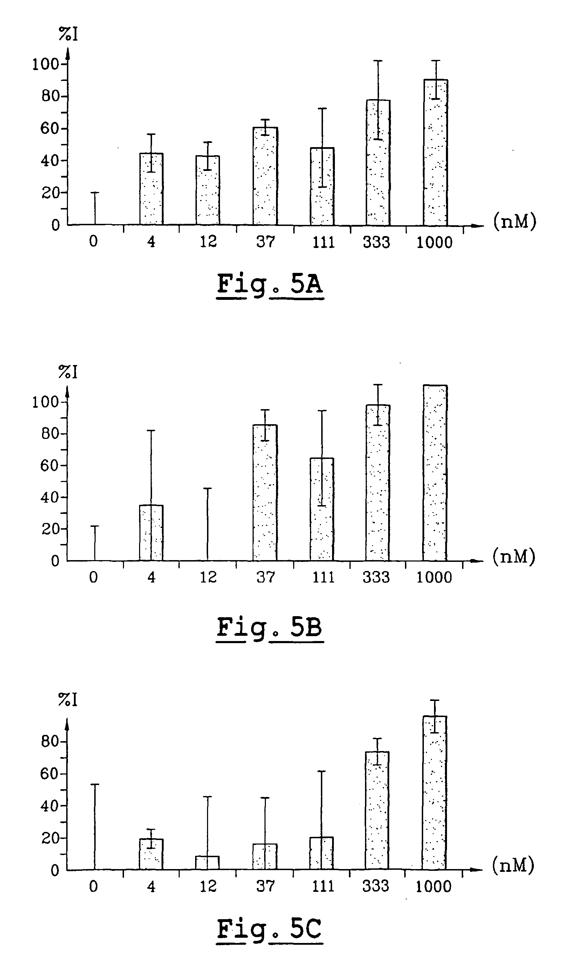

- Figures 5(A)-(C) : Effets des peptides CD4M30 (séquence ID n°10) (figure 4A), CD4M33 (séquence ID n°13) (figure 4B), CD4M35 (séquence ID n°14) (figure 4C) sur la réplication de la souche VIH-1$_{LAI}$ dans les CMSP activées par la PHA-P. Les données sont représentées en pourcentage d'inhibition (%I) de l'activité Transcriptase Inverse (TI) dans les surnageants de culture en fonction de la concentration en nM des peptides.

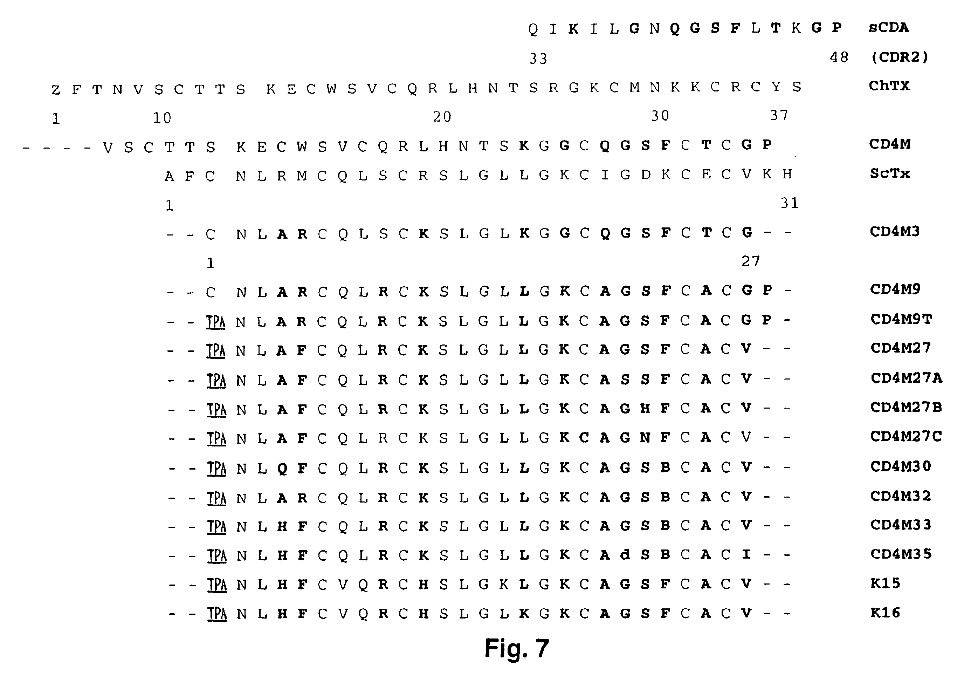

- Figures 6(A) et (B) : Effet de la présence du CD4 soluble et des peptides de la présente invention sur l'interaction de la protéine virale recombinante gp120$_{HXB2}$ avec les anticorps CG10 (A) et 48d (B), fixés à la surface d'une puce d'un instrument de résonance plasmonique de surface. Les courbes d'association (0-180 s) et de dissociation (180-400, 180-450 s) sont reportés pour la protéine virale gp120 seule et la protéine virale gp120 en présence du CD4M27 (1,5 équivalent, séquence ID n°6), du CD4M9 (1,5 équivalent), du mutant inactif (Ala23) CD4M9 (1,5 équivalent) et du CD4 soluble (1,5 équivalent). Les données sont reportées en unité de résonance (RU) en fonction du temps (t) en seconde.

- Figure 7 : Exemples de séquences peptidiques de la présente invention représentées avec les codes à une lettre des résidus acides aminés. TPA et B représentent l'acide thio-propionique et la bi-phénylalanine respectivement et "d" la forme optique D de l'acide aspartique.

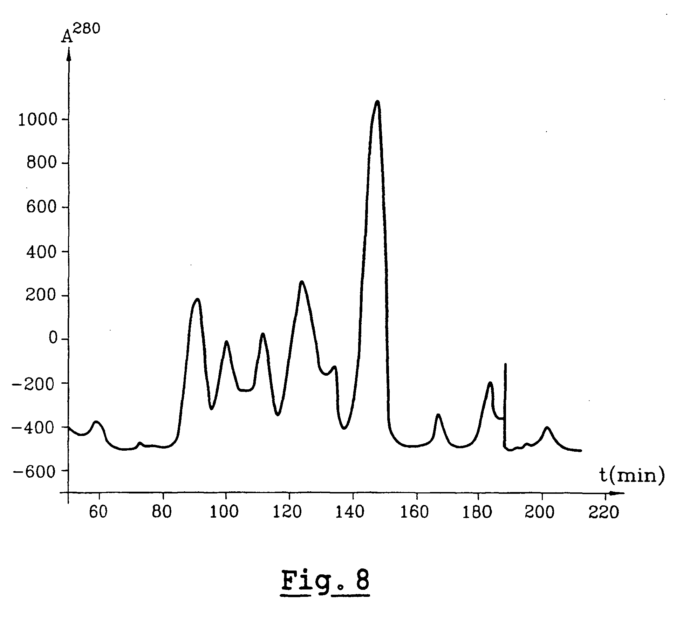

- Figure 8 : Profil chromatographique de la purification de la protéine recombinante gp120$_{HXB2}$ sur une colonne (1,2 x 4 cm) de Sepharose 4B fonctionnalisé avec le peptide de la présente invention CD4M9 (séquence ID n°3). Les données sont reportées en absorbance à 280 nm en fonction du temps (min), après addition de l'acide acétique 0,5 M.

## Exemples

### Exemple 1 : Synthèse des peptides de la présente invention.

Les peptides de la présente invention ont été fabriqués dans cet exemple par synthèse chimique en phase solide avec un synthétiseur automatique de peptides Applied Biosystems, mod. 433A, et en chimie Fmoc, qui utilise le groupement Fluorenylmethyloxycarbonyle (Fmoc) pour la protection temporaire de la fonction α-aminique des acides aminés. Les groupements protecteurs utilisés pour prévenir les réactions secondaires des chaînes latérales des acides aminés, dans cette stratégie Fmoc, ont été le tertio-butyle éther (tBu) pour les résidus Ser, Thr et Tyr ; tertio-butyle ester (OtBu) pour Asp, Glu; trityle (Trt) pour Gln, Asn, Cys, His ; tertio-butyloxycarbonyle (Boc) pour Lys et 2,2,5,7,8-pentametylchromane-6-sulfonyle (Pmc) pour Arg.

La réaction de couplage se déroule avec un excès de 10 équivalents d'acides aminés (1 mmol) par rapport à la résine (0,1mmol). L'acide aminé protégé est dissous dans 1 ml de N-methylpyrollidone (NMP) et 1 ml d'une solution de 1-N-hydroxy-7-azabenzotriazole (HOAt) 1M dans le solvant NMP. 1 ml d'une solution de N,N'-dicyclohexylcarbodiimide (DCC) 1M est alors ajouté. Après 40 à 50 minutes d'activation, l'ester actif formé est transféré dans le réacteur qui contient la résine. Avant cette étape de transfert puis de couplage, la résine est déprotégée de son groupement Fmoc par une solution de 20 % de pipéridine dans le NMP. L'excès de pipéridine est enlevé par lavage à la NMP après 5 à 10 minutes environ.

Pendant la déprotection, la détection des adduits dibenzofulvène-pipéridine à 305 nm permet de suivre le bon déroulement de la synthèse. En effet, la quantification de l'adduit permet d'estimer l'efficacité de la déprotection du groupement Fmoc et par suite du couplage du dernier acide aminé incorporé.

**Modifications chimiques apportées aux peptides de la présente invention**

Une sonde fluorescente ainsi qu'un groupement biotine ont été couplés sur deux des peptides de la présente invention étudiés, le CD4M9T (séquence ID n°4) et le CD4M33 (séquence ID n°13). L'incorporation sur ces deux composés s'est faite sur le résidu lysine 11, au niveau d'une face opposée au site de liaison de la protéine virale gp120. Ce choix d'une lysine a été conditionné par ses possibilités de protection de sa chaîne latérale avec un groupement protecteur, dit orthogonal, comme le groupement de (1-(4,4-diméthyl-2,6-dioxo-cyclohexylidène)éthyle). Un tel groupement est en effet stable au traitement à 20 % de pipéridine utilisé pour la déprotection du groupement Fmoc, mais est clivé de façon spécifique par traitement avec une solution d'hydrazine à 2 %. Une fois le groupement Dde retiré, la fluorescéine ainsi que la biotine ont pu être couplées.

Introduction de la fluorescéine. Le groupement fluorescent a été introduit sur la chaîne latérale de la lysine 11 du peptide de la présente invention CD4M33 et de la lysine 15 ou 16 des peptides K15 (séquence ID n°15) ou K16 (séquence ID n°16), respectivement, de la présente invention. Le groupement Dde a donc été utilisé pour la protection de la chaîne latérale de la lisine pendant la synthèse du peptide, et ensuite libéré par deux traitements de 5 min avec 2 % d'hydrazine dans la diméthylformamide (DMF). La molécule de fluorescéine a été couplée directement sur le peptide synthétisé ou par l'intermédiaire d'un bras, qui sert à éloigner la fluorescéine, molécule encombrante, ce qui pourrait gêner l'activité de liaison. Dans ce dernier cas, la molécule permettant d'introduire un bras est l'acide Fmoc-8-amino-3,6-dioxaoctanoïque. A un équivalent de résine sont ainsi ajoutés, 17 équivalents d'acide Fmoc-8-amino-3,6dioxaoctanoïque, 60 équivalents de diisopropyléthylamine (DIEA), 17 équivalents de hexafluorophosphate de 2-(1H-benzotriazol-1-yl)-1,1,3,3-tetramethyluronium (HBTU). Après couplage pendant 1 heure à température ambiante, le groupement Fmoc est déprotégé avec la pipéridine 20 %. Le couplage de la sonde a ensuite été réalisé à l'aide de l'ester fluorescéine-(5-(6)-carboxylate de N-hydroxysuccinimidyle. Pour cela, à un équivalent de résine sont ajoutés 4 équivalents d'ester activé de fluorescéine en présence de 14 équivalents de diisopropyléthylamine (DIEA). La réaction se déroule dans le solvant NMP pendant une nuit. La déprotection finale est enfin réalisée.

Introduction de la biotine. Un bras espaceur 8-amino-3,6dioxaoctanoïque a été ajouté dans un premier temps sur la lysine 11 préalablement déprotégé du groupement Dde. La réaction est similaire à celle décrite dans le paragraphe précédent. La biotine est ensuite incorporée sous la forme d'un ester biotinamidocaproate de N-hydrosuccinimidyle : à un équivalent de résine sont ainsi ajoutés 4 équivalents d'ester activé de biotine en présence de 14 équivalents de diisopropyléthylamine (DIEA). La réaction se poursuit pendant une nuit à température ambiante. La résine est ensuite lavée avant d'être traitée par la solution de déprotection finale.

Introduction d'un groupement thiol. Le peptide CD4M9 est synthétisé selon la procédure décrite ci-dessus avec la Lysine en position 11 présentant comme groupement protecteur de la chaîne latérale un groupement Dde. Après synthèse du peptide, le peptide-résine est traité à 5 reprises avec une solution de 2% d'hydrazine dans la DMF. Le couplage d'un bras de liaison est réalisé pendant une heure à température ambiante dans la DMF avec 10 équivalents d'acide Fmoc-8-amino-3,6-dioxaoctanoïque utilisant le réactif HBTU en présence de diisopropyléthylamine. Le groupement Fmoc est ensuite déprotégé avec 20% de pipéridine dans la DMF. Le peptide-résine est dès lors traité avec 10 équivalents de réactif de Traut (hydrochlorure de 2-iminothiolane (Sigma) en présence de DIEA. Le peptide est enfin libéré et déprotégé comme décrit ci-dessous.

**Déprotection finale de la résine**

Le clivage de la résine et des groupements protecteurs présents sur les chaînes latérales ont été réalisées simultanément par traitement du peptide lié à la résine par de l'acide trifluoroacétique (TFA). Avant d'effectuer le clivage, la résine a été lavée plusieurs fois au dichlorométhane (DCM) et enfin séchée. Le réactif utilisé lors du clivage est un mélange acide contenant 81,5 % de TFA et les piégeurs phénol (5 %), thioanisole (5 %), eau (5 %), ethanedithiol (2,5 %) et tri-isopropylsilane (1 %). La résine a été traitée avec ce mélange pendant trois heures sous agitation et à température ambiante, à raison de 100 ml de solution par gramme de résine. Le peptide libre en solution a été récupéré par filtration. Le peptide a été ensuite précipitée et lavée à froid dans l'éther de diisopropyle puis dissous dans de l'acide acétique à 20 % et lyophilisée.

**Repliement des peptides de la présente invention par formation de ponts disulfure**

Le peptide récupéré après lyophilisation, le brut de synthèse, se trouve sous forme réduite, c'est-à-dire que les ponts disulfure intrachaînes ne sont pas formés. La formation de ces liaisons covalentes a été réalisée en utilisant le couple redox cystamine/cystéamine. Le brut de synthèse a été repris dans l'eau ajoutée de TFA 0,1% (v/v) et de chlorure de guanidinium 6M pour faciliter sa dissolution, à raison de 2,0 mg.ml$^{-1}$. Cette solution a ensuite été ajoutée au goutte-à-goutte, diluée à O,2 mg/ml$^{-1}$, au tampon de réduction, composé de Tris/HCl 100mM, pH 7,8, et de cystéamine 5 mM. La cystamine (oxydant) 0,5 mM en final, a été ajoutée après 45 minutes de réaction à température ambiante. Le milieu est amené à pH 3,0 après 30 minutes.

La cystéamine permet de réduire les groupements thiols présents sur le peptide. A l'air libre, elle s'oxyde et permet l'oxydation de cystéines et donc le repliement du peptide par formation de ponts disulfure intrachaînes. La cystamine ajoutée en fin de manipulation permet de parfaire le repliement. Le bon déroulement de l'oxydation est vérifié par chromatographie analytique en comparant les temps de rétention des produits brut et oxydé, plus importants pour le premier.

L'oxydation du peptide CD4M9, présentant un groupement thiol en position 11, diffère quelque peu de celle décrite dans le paragraphe plus haut. Le milieu de repliement réactionnel, composé d'un tampon 20 mM phosphate, 200 mM NaCl, ajusté à pH 7,8 est longuement dégazé par de l'argon et ensuite ajouté de 5 mM de cystéamine, 5 mM cytamine. Le peptide comprenant sept fonctions SH libres est ensuite ajouté et la réaction d'oxydation est stoppée par ajout d'acide après seulement 10 minutes, temps suffisant au repliement et à la formation des trois ponts disulfure naturels et suffisamment court pour limiter la formation de produit secondaire correspondant à des états d'oxydation supérieurs.

**Purification des peptides de la présente invention**

Les peptides de la présente invention ont été purifiés par chromatographie liquide haute performance en phase inverse sur une colonne préparative Vydac C18 (1,0 x 25,0 cm). On a utilisé un gradient linéaire 0-60 % d'acétonitrile dans une solution aqueuse d'acide trifluoroacétique à 0,1 % pendant 90 minutes. Les fractions du pic majeur ont été analysées par HPLC analytique; les fractions ne présentant qu'un seul pic ont été rassemblées et lyophilisées. Le produit ainsi obtenu a été analysée par spectrométrie de masse.

**Exemple 2 : Tests de Compétition par ELISA indirecte.**

L'inhibition de l'interaction rgp120-CD4 a été mesurée par ELISA (Enzyme Linked ImmunoSorbent Assay) indirecte. 50 ng par puits d'anticorps anti-gp120, D7324, ont été immobilisés sur une plaque 96 puits (Maxisorb, Nunc) pendant une nuit à 4°C. Après passivation par la Sérumalbumine Bovine et trois lavages avec le tampon de lavage (Tris 10 mM, pH 7,8, Tween 20 0,05 %), 15 ng de rgp120$_{HXB2}$ par puits ont été ajoutés. Différentes concentrations de compétiteurs ont ensuite été ajoutées, après trois lavages, ainsi que 0,4 ng de CD4 soluble par puits. Des témoins 100 % ne contenant que du CD4 soluble sont effectués. Après une nuit à 4°C et trois lavages, 5 ng d'anticorps de souris anti-CD4 L120.3 ont été ajoutés par puits, puis un anticorps de chèvre anti-IgG de souris couplé à la peroxydase (GAMPO). La révélation a été effectuée par ajout de 3,3',5,5'-tétraméthylbenzidine, substrat fluorescent de la peroxydase. La réaction a été arrêtée après 30 minutes par ajout d'acide sulfurique 2M. L'inhibition de l'interaction rgp120-CD4 a été calculée par lecture de l'absorbance A à 450 nm. Des témoins sans compétiteur ont permis de déterminer l'absorbance $A_{100}$% (absorbance en absence de compétiteur) . Le pourcentage d'inhibition pour chaque concentration de peptide de la présente invention a été calculé par la formule:

$$\text{pourcentage d'inhibition} = 100 \times (A_{100\%}-A)/A_{100\%}.$$

Les tests ont été conduits en duplicats et les résultats exprimés comme la moyenne de doublons expérimentaux.

Les activités biologiques du peptide CD4MO (séquence ID n°18) issu de la charybdotoxine et du peptide CD4M3 (séquence I.D. n°17), issu de la scyllatoxine (séquence I.D. n°2) ont été testées en test ELISA indirecte. Ces peptides présentent une capacité à inhiber l'interaction rgp120$_{LAI}$-CD4 soluble, avec une concentration d'inhibition à 50% (ou CI$_{50}$) de $4,0 \times 10^{-5}$ M et de $2,0 \times 10^{-5}$ M, respectivement (Tableau I) [4]. Ces valeurs sont 10 000 fois plus élevées que la CI$_{50}$ présentée par le CD4 soluble (CI$_{50}$ = $4,0 \times 10^{-9}$ M) [4].

La figure 1 annexée regroupe les résultats obtenus dans cet exemple avec les peptides de la présente invention.

Il s'agit de représentations du pourcentage de fixation (%F) de la protéine virale gp120$_{HXB2}$-CD4 en fonction de la concentration molaire (C(M)) en compétiteur, c'est-à-dire en peptide de la présente invention. Cette figure montre l'inhibition de l'interaction CD4-gp120$_{HXB2}$.

Le CD4M9 (séquence ID n°3) a été testé en ELISA par compétition. Il présente la capacité d'inhiber l'interaction rgp120$_{LAI}$-CD4 soluble avec une concentration d'inhibition à 50% (ou CI$_{50}$) de $4,10^{-7}$ M (Tableau I) soit une augmentation de la capacité d'inhibition d'un facteur 50 par rapport au peptide de la présente invention CD4M3 (séquence ID n°17). Le peptide de la présente invention CD4M9, en test ELISA par compétition, est également capable d'inhiber l'interaction du CD4 avec d'autres protéines gp120 recombinantes, provenant de virus T- et M-tropique : VIH-1$_{IIIB}$, VIH-1$_{MN}$, VIH-1$_{Ba-L}$, VIH-1$_{JR-FL}$, VIH-1$_{W61D}$, avec une concentration d'inhibition à 50% (CI$_{50}$) entre 0,1 et $1,0 \times 10^{-6}$ M [4].

Ce peptide inhibe l'interaction gp120$_{HXB2}$-CD4 soluble à une CI$_{50}$ de $9 \times 10^{-8}$M (figure 1, tableau I).

Un mutant CD4MV, comportant la substitution de la séquence C-terminale Gly-Pro du CD4M9 par Val, présente une $CI_{50}$ de $3,0.10^{-7}$ M dans l'inhibition de l'interaction $gp120_{LAI}$-CD4 (tableau 1), ce qui représente une légère augmentation de la capacité inhibitrice. Le mutant CD4M9Bip comporte la substitution Phe23Bip par rapport au CD4M9 ; ce peptide présente une $CI_{50}$ de $1,0.10^{-7}$M dans l'inhibition de l'interaction $gp120_{LAI}$-CD4 (tableau 1), ce qui représente une augmentation supplémentaire de la capacité inhibitrice. Le mutant CD4M9T (séquence ID n°4), comportant un acide thio-propionique en substitution de l'acide aminé Cys N-terminal, présente une $CI_{50}$ de $3,0.10^{-8}$ M dans l'inhibition de l'interaction $gp120_{LAI}$-CD4 (figure 1; la $CI_{50}$ dans l'interaction $gp120_{HXB2}$ est de $1,1.10^{-8}$ M, tableau 1), ce qui représente une augmentation de la capacité d'inhibition d'un facteur proche de 10 par rapport à CD4M9.

Les mutants suivants présentent tous un résidu thio-propionique (TPA) et valine (Val ou Ile) en position N- et C-terminale, respectivement, et ont tous été testés avec la gp120, issue des virus T tropiques $VIH-1_{HXB2}$ et $VIH-1_{LAI}$.

Le peptide de la présente invention CD4M27 (séquence I.D. n°6) comporte les mutations Arg5Phe (pour enlever une arginine non essentielle et protéger le pont disulfure 6-24), Gly27Val et la délétion du résidu C-terminal proline (pour mieux stabiliser la structure β). Il présente une augmentation de la capacité d'inhibition de la liaison CD4 - $gp120_{HXB2}$, $CI_{50}$ de $7.10^{-9}$ M, (Fig. 1, Tableau I). Les peptides CD4M27 A, B et C sont obtenus respectivement par une mutation Gly21Ser(a), Ser22His(b) et Ser22Asn(c) à partir du peptide CD4M27. Ils présentent une capacité d'inhibition comparable à celle du peptide CD4M27.

Le peptide de la présente invention CD4M32 (séquence ID n°12), comparée au CD4M9T, comporte la délétion du résidu proline C-terminal, la mutation Gly27Val et la mutation de la Phe23 en biphenylalanine (Bip) : la mutation Phe23Bip ajoute une extension hydrophobe à la chaîne latérale de la Phe23, pour augmenter l'interaction avec la cavité hydrophobe de la protéine virale gp120. CD4M32 présente une augmentation de l'inhibition de la liaison CD4-$gp120_{HXB2}$ ($CI_{50}$ de $8.10^{-10}$ M, Fig. 1). Le mutant CD4M30 (séquence ID n°10), par rapport au CD4M32, comporte la mutation Arg5Phe (déjà introduite dans le CD4M27) et Ala4Gln pour augmenter la solubilité de la molécule. Ce peptide de la présente invention présente un $CI_{50}$ de 3,0 nM, dans les tests d'inhibition de l'interaction CD4 - $gp120_{LAI}$. Le mutant CD4M33 (séquence ID n°13) regroupe les mutations présentes dans les deux peptides précédents, notamment Cys1TPA, Arg5Phe, Phe23Bip, Gly27Val, la délétion d'un résidu en position C-terminale et en plus la mutation Ala4His (pour augmenter la solubilité de la molécule). Ces mutations ont un effet additif et permettent .au peptide de la présente invention CD4M33 d'inhiber l'interaction CD4- $gp120_{HXB2}$ et CD4-$gp120_{LAI}$ avec une $CI_{50}$ de $1,2 \times 10^{-10}$ M et de $2,5 \times 10^{-10}$ M (Fig. 1 et Tableau 1) soit une augmentation de la capacité d'inhibition de la liaison CD4 - gp120 d'un facteur 1000 par rapport au CD4M9. Un peptide supplémentaire, CD4M35 (séquence ID n°14), a été synthétisé avec, par rapport au peptide CD4M33 de la présente invention, les mutations Va127Ile et Gly21(D)Asp. Ces mutations permettent au peptide de la présente invention d'inhiber l'interaction CD4-$gp120_{HXB2}$ avec un $CI_{50}$ de $7.0 \; 10^{-11}$ M (Fig. 1, Tableau 1).

Deux autres peptides K15 (séquence ID n°15) et K16 (séquence ID n°16) ont été synthétisés : ils comportent les substitutions Gln7Val, Leu8Gln, Lys11His, par rapport au peptide CD4M33 de la présente invention, et un résidu Lys en position 15 ou 16, respectivement. Un groupement fluorescent de fluorescéine a ensuite été fixé, d'une façon covalente, au niveau de la chaîne latérale de ce résidu lysine, selon le protocole décrit dans l'exemple 1. Les peptides fluorescents K15 et K16 présentent une $CI_{50}$ de $5,0.10^{-8}$ M et $6,0.10^{-9}$M dans l'inhibition de l'interaction $gp120_{LAI}$-CD4, dont l'affinité pour la protéine virale gp120 n'est pas modifiée par un marquage.

En conclusion, la présente invention fournit une famille de peptides qui représentent de puissants inhibiteurs de l'interaction CD4-gp120.

**Tableau 1**

| Concentration d'inhibition 50% ($CI_{50}$) des peptides mimétiques du CD4 dans l'interaction du CD4 recombinant soluble pour la protéine de l'enveloppe virale $gp120_{LAI}$ et $gp120_{HXB2}$. L'écart type pour chaque valeur est inférieur à 30%. | | | |
|---|---|---|---|
| Nom | Séquence IDn° | $CI_{50}$ ($gp120_{LAI}$) | $CI_{50}$ ($gp120_{HXB2}$) |
| CD4M0 | 18 | 40 μM | - |
| CD4M3 | 17 | 20 μM | - |
| CD4M9 | 3 | 400 nM | 90 nM |
| CD4M9V | - | 300 nM | - |
| CD4M9T | 4 | 30 nM | 11 nM |
| CD4M9Bip | - | 100 nM | - |
| CD4M27 | 6 | 10 nM | 7 nM |
| CD4M30 | 10 | 3,0 nM | - |
| CD4M32 | 12 | 2,0 nM | 800 pM |
| CD4M33 | 13 | 250 pM | 120 pM |
| CD4M35 | 14 | - | 70 pM |

(suite)

| Concentration d'inhibition 50% (CI$_{50}$) des peptides mimétiques du CD4 dans l'interaction du CD4 recombinant soluble pour la protéine de l'enveloppe virale gp120$_{LAI}$ et gp120$_{HXB2}$. L'écart type pour chaque valeur est inférieur à 30%. | | | |
|---|---|---|---|
| Nom | Séquence IDn° | CI$_{50}$ (gp120$_{LAI}$) | CI$_{50}$ (gp120$_{HXB2}$) |
| K15FR | 15 | 50 nM | - |
| K16FR | 16 | 6 nM | - |

## Exemple 3 : Expériences de résonance plasmonique de surface

Les expériences de résonance plasmonique de surface ont été réalisées avec un système Biacore 2000 (Biacore, Uppsala, Suède). Les peptides à tester ont été couplés à la biotine (comme décrit auparavant) puis immobilisées sur une puce sur laquelle de la streptavidine a été préalablement fixée.

La streptavidine a été immobilisée sur la puce comme suit: la surface de la puce a été d'abord activée par l'injection de 50 $\mu$L d'agent de couplage prévu par le constructeur et pouvant former des liaisons amidiques : N-ethyl-N'-(dimethylaminopropyl) carbodiimide (EDC)/N-hydroxysuccinimide (NHS), 50/50 ; ensuite, 20 $\mu$L de streptavidine, 0,2 mg.mL$^{-1}$ dans l'acétate de sodium 10 mM, pH 4,5, ont été injectés à 5 $\mu$L. min$^{-1}$, suivis d'une neutralisation des groupements carboxyliques activés par 2 x 20 $\mu$L d'éthanolamine 1,0 M, pH 8,5. Les molécules biotinylées ont été ensuite injectées (10 $\mu$L.min$^{-1}$ dans un tampon Hepes 10 mM, NaCl 0,3 M, pH 7,4) sur trois des quatre pistes de la puce. Sur la première piste, le CD4M9 (10$^{-7}$ M) a été immobilisé jusqu'à une valeur RU (unité de résonance) 100; la deuxième piste a été laissée vierge (témoin) ; la troisième piste a été recouverte avec du CD4 soluble (10$^{-7}$ M) jusqu'à une valeur de 450 RU ; sur la quatrième piste, le CD4M33 (10$^{-7}$ M) a été immobilisé à une valeur de 100 RU. Pour chaque analyse, plusieurs concentrations de différentes gp120 (souches HXB2, BAL, JRFL) ont été injectées, à une vitesse de 50 $\mu$L.min$^{-1}$ à 25°C, pour un temps d'association de 4 min. La puce est ensuite rincée avec un tampon de course, 10 mM Hepes, 150 mM NaCl, 3,4 mM EDTA et 0,05 % P20, pH 7,4, pour analyser la phase de dissociation. Après chaque expérience, la puce est régénérée avec 25 $\mu$L d'acide formique 1,0 M. Les constantes de dissociation sont calculées à partir des constantes cinétiques déterminées par le logiciel Bia-evaluation 3,0.

## Exemple 4 Activité antivirale des mimétiques CD4.

Les manipulations du matériel infectieux ont été réalisées dans un laboratoire de haute sécurité de type L3. De façon à être le plus proche des conditions physiopathologiques, l'étude a été menée à l'aide de cultures primaires de cellules mononucléées du sang périphérique (CMSP) humaines. Dans toutes les expériences, les effets des nouvelles molécules ont été comparés à ceux de l'AZT.

Isolement culture et activation des cellules Milieux de culture

Le milieu A est composé de milieu de culture cellulaire RPMI 1640 (Life Technologies) supplémenté par 10% de sérum de veau foetal (SVF, Roche Product) décomplémenté par la chaleur à +56°C pendant 30 min, de 2 mM de L-Glutamine (Roche Product), et d'une solution à 100 $\mu$g/ml de trois antibiotiques (pénicilline, streptomycine, néomycine ; PSN, Life Technologies). Le milieu B est constitué de milieu A supplémenté par 20 UI/ml d'IL-2 humaine recombinante (Roche Product).

Isolement et activation des CMSP

Les CMSP sont séparées des autres éléments figurés du sang par centrifugation en gradient de ficoll (MSL 2000, Eurobio): 30 ml de sang, d'un donneur sain, dilués au tiers sont déposés sur un coussin de 20 ml de ficoll. Après 20 min de centrifugation à 850 g, l'anneau de CMSP est prélevé puis lavé deux fois à l'aide de RPMI 1640, après 10 min de centrifugation à 750 g et 5 min à 400 g. Les CMSP sont alors activées pendant 48 h par 1 $\mu$g/ml de phytohémagglutinine-P (PHA-P ; Difco Laboratories). Les CMSP sont cultivées à +37°C, en atmosphère saturée en humidité, sous 5% de CO$_2$. Au terme des 48 heures d'activation mitogénique, elles sont cultivées en milieu B. Tout au long de la culture, les surnageants de culture sont prélevés, et les milieux de culture sont renouvelés tous les trois ou quatre jours. A chaque renouvellement des milieux de culture, la viabilité cellulaire est évaluée par une observation microscopique.

Evaluation de l'activité antirétrovirale des molécules

Les composés de la présente invention CD4M30, CD4M33 et CD4M35 ont été solubilisés dans de l'eau ppi stérile (Aguettant), aliquotés puis conservés à -20°C. Le composé SAH-CD4 (CD4 soluble couplé à la sérum albumine humaine fournit par Aventis (Vitry-sur-Seine) a été conservé à -80°C jusqu'à son utilisation. Les solutions et les dilutions ont ensuite été réalisées extemporanément dans du milieu A. Les CMSP ont été prétraitées avec les composés pendant 1 heure puis infectées par l'isolat de référence à tropisme lymphocytaire VIH-1$_{LAI}$ ou par l'isolat clinique VIH-1$_{LEN}$. Les caractéristiques biologiques de cet isolat sont : rapid/high, scyncitia inducing (SI), X4 : il est donc préférentiellement apte à infecter les lymphocytes. Le stock viral a été constitué en amplifiant *in vitro* cette souche à l'aide de cellules mononucléées du sang ombilical (CMSO) activées préalablement par 1 μg/ml de PHA-P et cultivées dans du milieu B. Afin d'éliminer les facteurs solubles tels que les cytokines, les surnageants de culture ont été ultracentrifugés à 360 000 g pendant 5 min, et les culots ont été resuspendus dans du RPMI 1640. Le stock viral ainsi constitué a été ensuite titré à l'aide de CMSP activées par la PHA-P. La TCID$_{50}$ (50% Tissue Culture Infectious Dose) a été calculée en utilisant la formule de Kärber. Les CMSP ont été infectées avec différentes doses virales 10-100 TCID$_{50}$ de la souche VIH-1$_{LAI}$ et par 50 TCID$_{50}$ de la souche VIH-1$_{LEN}$ (multiplicité d'infection m.o.i. = 0,001).

Dosage de la réplication virale dans les surnageants de culture.

La réplication virale a été mesurée, au jour 7 de la culture, en dosant l'activité Transcriptase Inverse dans les surnageants de culture à l'aide de la trousse de dosage RetroSys (marque déposée) selon les recommandations de la société Innovagen.

Analyse des résultats et détermination des doses effectrices 50%

Les doses effectrices 50% (DE$_{50}$) sont calculées en utilisant le logiciel "Dose-effects analysis with microcomputers" mis au point par J. Chou & T.C. Chou.

## Exemple 5 : Protocole de production de la protéine virale gp120 recombinante.

Le fragment codant la protéine virale gp120 (acide aminé V12 à R481) a été amplifié par PCR dans le plasmide HIVIIIB/HXB2R, à l'aide de deux amorces permettant de générer un fragment contenant les sites BamHI (en amont du site KpnI de la protéine virale gp120) et PstI (en aval du codon stop ajouté à l'extrémité de la séquence de la protéine virale gp120). Le fragment BamHI/PstI ainsi obtenu a été cloné dans le vecteur Bluescript (pBS, Stratagene), conduisant au plasmide pBSm1. La séquence codant l'extrémité N-terminale de la protéine virale gp120 (T1 à G11), ainsi que celle codant le peptide signal de l'ecdystéroïde glycosyltransférase du baculovirus d'*Autographa californica,* ont été insérées entre les sites BamHI et KpnI de pBSm1, conduisant à pBSm2.

Le fragment BamHI-PstI de pBSm2 a ensuite été inséré entre les sites BglII-PstI du vecteur de transfert p119P du baculovirus P10. Des cellules d'insectes Sf9 ont été cotransfectées avec l'ADN viral purifié du baculovirus modifié AcSLP10 et l'ADN du vecteur recombinant pll9P gp120. Les virus recombinants sont purifiés par une méthode standard.

Les cellules Sf9 scla (lignée adaptée à la croissance sans sérum, déposée à la collection de l'Institut Pasteur) sont entretenues en spinner en milieu sans sérum. Ces cellules (5.10$^5$ cellules/mL) sont ensuite infectées par les virus recombinants à une multiplicité d'infection de 1 UFP (Unité Formant Plage) par cellule et incubées à 28°C. Après six jours d'infection, les cellules sont centrifugées (500 g), et la protéine virale gp120 sauvage est concentrée et directement purifiée à partir du surnageant de culture par chromatographie d'affinité sur une colonne de Sépharose-bromacétylée couplée à l'anticorps anti-gp120 D7324.

## Exemple 6 Fixation de l'enveloppe virale.

Afin de mieux caractériser son activité biologique, le peptide de la présente invention CD4M33 (séquence ID n°13) a été marqué avec la biotine et avec la sonde fluorescente fluorescéine comme décrit dans l'exemple 1, au niveau de la Lys-11 positionnée sur la surface opposée de la surface active du peptide de la présente invention CD4M33. Son activité demeure inchangée dans des tests ELISA (Figure 2).

La figure 2 regroupe les résultats obtenus dans cet exemple. Il s'agit de courbes montrant l'évolution du pourcentage de fixation (%F) de peptides de la présente invention à la protéine virale gp120$_{HXB2}$ en fonction de la concentration molaire de ces peptides (C(M)).

Ces résultats montrent que le peptide CD4M33 peut incorporer un groupement de marquage sans que son activité biologique soit altérée.

Afin d'obtenir une première évaluation de l'affinité du peptide de la présente invention CD4M33 pour la protéine virale gp120, une analyse d'interactions biospécifiques (décrite dans l'exemple 3), basée sur la détection par résonance plasmonique de surface, a été utilisée. Dans cette technique, le peptide CD4M33 de la présente invention biotinylé (préparé comme dans l'exemple 1) a été fixé de façon spécifique sur la matrice de dextran carboxylé d'une puce qui a été prégreffé avec de la streptavidine. Des solutions de différentes protéines recombinantes gp120 (gp120$_{HXB2}$, gp120$_{BAL}$ et gp120$_{JRFL}$) ont été ensuite injectées sur cette matrice et un signal indiquant une association spécifique et de haute affinité a été alors détecté. La figure 3 montre l'évolution du signal de résonance plasmonique en fonction du temps, après interaction de la protéine gp120$_{HXB2}$ (à des concentrations différentes) avec le peptide CD4M33 de la présente invention.

Après régression non-linéaire des courbes d'association et de dissociation obtenues, on obtient une constante de dissociation K$_D$ de 2.4.10$^{-9}$ M pour la protéine virale gp120$_{HX32}$, de 7.4.10$^{-9}$ M pour la protéine virale gp120$_{BAL}$ et de 8.5.10$^{-9}$ M pour la protéine virale gp120$_{JRFL}$. Ces valeurs sont comparables à la valeur de K$_D$=1,9×10$^{-8}$ M, reportée dans la littérature [34] pour l'interaction CD4-gp120. La même technique basée sur la détection par résonance plasmonique de surface a été aussi utilisée pour vérifier si le peptide de la présente invention CD4M33 pouvait fixer l'enveloppe virale dans sa forme native. Pour ce faire, une suspension de particules virales inactivées par l'adrithiol-2 [35] a été injectée sur la même puce, greffé avec les anticorps 48d et CG10. Un signal d'interaction spécifique et de haute affinité a été alors clairement détecté, dans le cas où la suspension était incubée avec le peptide de la présente invention CD4M33, mais pas dans le cas où la suspension virale était incubée avec le peptide CD4M9, beaucoup moins actif, ou en absence du peptide (figure 4).

La figure 4 montre que la présence du peptide CD4M33 est essentielle pour l'interaction de l'enveloppe virale avec les anticorps 48d et CG10, spécifiques de l'enveloppe virale, et que le VIH-1 ne se fixe pas aux anticorps en son absence : il s'agit d'une démonstration indirecte de la fixation du peptide CD4M33 à l'enveloppe virale du VIH-1.

Ces expériences démontrent que le peptide de la présente invention CD4M33 marqué ou non possède une capacité à fixer l'enveloppe virale VIH tant dans sa forme recombinante isolée et purifiée que dans sa forme native présente à la surface du virus.

### Exemple 7 Inhibition de l'infection par VIH-1

Afin d'évaluer la capacité antivirale des peptides de la présente invention, les inventeurs ont conduit des expériences d'infection, par le virus VIH-1$_{LAI}$ et par l'isolat chimique VIH-1$_{LEN}$, de cultures primaires de cellules mononucléées du sang périphérique (CMSP) humaines. Dans toutes les expériences, les effets des nouvelles molécules ont été comparés à ceux de l'AZT. Le virus VIH-1$_{LAI}$ a été ajouté à des différentes doses virales (10-100 TCID$_{50}$, Tableau 2) en présence de concentrations variables de CD4M30, CD4M33, CD4M35 et du SAH-CD4. Le virus VIH-1$_{LEN}$ a été ajouté à TCID$_{50}$ (tableau 4) en présence de CD4M33. Des tests de toxicité de mimétiques du CD4 ont été aussi effectuées sur les mêmes cellules.

a. Toxicité des mimétiques CD4. Aux doses testées, aucun des composés, AZT, SAH-CD4 ou mimétiques anti-CD4, n'a diminué la viabilité des CMSP activées par la PHA-P.

b. Activité anti-VIH-1$_{LAI}$ des mimétiques CD4.

b.1. Réplication de la souche VIH-1$_{LAI}$ dans les CMSP. La souche VIH-1$_{LAI}$ réplique à haut niveau dans les CMSP activées par la PHA-P. Le pic de réplication virale est au jour 7 de la culture, et les effets des peptides CD4M30, CD4M33 et CD4M35 ont été quantifiés à ce temps post-infection.
b.2. Effets de l'AZT sur la réplication de la souche VIH-1$_{LAI}$ dans les CMSP. L'AZT inhibe fortement la réplication de la souche VIH-1$_{LAI}$ dans les CMSP activées par la PHA-P (Tableau 2). La DE$_{50}$ est égale à 3-14 nM.
b.3. Effets du composé SAH-CD4 sur la réplication de la souche VIH-1$_{LAI}$ dans les CMSP. L'activité antirétrovirale du composé SAH-CD4 vis-à-vis des CMSP activées par la PHA-P et infectées par la souche VIH-1$_{LAI}$ se traduit par une inhibition de 85±15% de la réplication virale à la concentration de 2,5 μM.
b.4. Effets des mimétiques CD4 sur la réplication de la souche VIH-1$_{LAI}$ dans les CMSP. Les peptides de la présente invention CD4M30, CD4M33 et CD4M35 ont démontré une activité anti-rétrovirale (Tableau 3) dans les cultures de CMSP activées par la PHA-P et infectées par la souche VIH-1$_{LAI}$. Le composé CD4M33 est le plus antiviral des trois aux différentes doses virales testées. Son activité est plus faible de celle de l'AZT : DE$_{50}$ = 100-500 nM vs. AZT : DE$_{50}$ = 3 nM (tableau 3 ci-dessous), mais elle est supérieure d'au moins 1 logarithme de base 10 à celle du dérivé du récepteur CD4, SAH-CD4.

Les figures 5A-C regroupent les résultats obtenus : pourcentage d'inhibition (%I) en fonction de la concentration en peptide en nM.

c. Activité antirétrovirale du composé CD4M33 vis-à-vis de l'isolat clinique VIH-1$_{LEN}$

c1. Effets de l'AZT sur la réplication de l'isolat VIH-1$_{LEN}$ dans les CMSP

L'AZT inhibe fortement la réplication de la souche VIH-1$_{LEN}$ dans les CMSP activées par la PHA-P et infectées par l'isolat VIH-1$_{LEN}$, avec une DE$_{50}$ égale à 2,2 nM (tableau 4). Le degré d'inhibition est identique à celui observé vis-à-vis de la souche de référence à tropisme lymphocytaire VIH-1$_{LAI}$.

c2. Effets du mimétique CD4M33 sur la réplication de l'isolat clinique VIH-1$_{LEN}$ dans les CMSP

L'activité rétrovirale du mimétique CD4M33 vis-à-vis des CMSP activées par la PHA-P et infectées par l'isolat VIH-1$_{LEN}$ se traduit par une diminution dose dépendante de la réplication virale et une DE$_{50}$ égale à 367 nM (tableau 4). Le mimétique CD4M33 conserve donc une activité anti-VIH-1 significative, même si elle s'avère légèrement plus faible par rapport à celle observée avec la souche VIH-1$_{LAI}$.

Ces expériences démontrent que ces peptides de la présente invention sont capables d'inhiber l'infection des cellules, même à des doses virales élevées, avec des valeurs de DE$_{50}$ de 900-35 nM (Tableau 3 ci-dessous). Le peptide de la présente invention CD4M33 est le plus antiviral et son DE$_{50}$ est d'environ 100 nM pour les doses virales standards d'infection (Fig. 4 a-c, Tableau 3 ci-dessous).

En outre, le peptide CD4M33 est doté d'une activité antirétrovirale supérieure à 1 logarithme de base 10 à celle observée avec un autre dérivé du récepteur CD4, le SAH-CD4, et surtout a montré une activité anti-VIH significative vis-à-vis d'un isolat clinique (tableau 4).

En conclusion, le peptide de la présente invention CD4M33, qui est capable de se fixer sur la protéine virale gp120 recombinante avec une Kd de 2.4-8.0 nM (expériences de résonance plasmonique de surface) et d'inhiber l'interaction entre les protéines recombinantes CD4-gp120 en ELISA, avec une CI$_{50}$ de 120-250 pM, présente aussi la capacité d'inhiber cette interaction dans des cultures primaires de cellules humaines, d'inhiber l'étape initiale de l'entrée et donc de bloquer l'infection même d'un isolat clinique VIH-1.

**Tableau 2**

| Effet de l'AZT sur la réplication de la souche VIH-1$_{LAI}$ dans les CMSP activées par la PHA-P. Les résultats sont exprimés en pourcentages d'inhibition + écart-type . | |
|---|---|
| AZT (nM) | % Inhibition |
| 1 | 39+ 10% |
| 10 | 59+28% |
| l00 | 87$\pm$18% |
| 100 | 100$\pm$0% |
| 10 000 | 100$\pm$0% |

**Tableau 3**

| Doses effectives 50% (DE$_{50}$) des peptides de la présente invention et de l'AZT dans des cultures de cellules mononucléées du sang périphérique (CMSP) humaines, activées par la phytohémagglutinine-P (PHA-P) et infectées par la souche VIH-1$_{LAI}$ à des différentes doses infectieuses (TCID$_{50}$: 50% Tissue Culture Infectious Dose) : | | | |
|---|---|---|---|
| **DE$_{50}$ (nM)** | | | |
| séquences | 100 TCID$_{50}$ | 50 TCID$_{50}$ | 10 TCID$_{50}$ |
| CD4M30 | 894 | 235 | 253 |
| CD4M33 | 534 | 133 | 118 |
| CD4M35 | 154 | 428 | 266 |
| AZT | 14 | 3 | 5 |

# EP 1 368 372 B1

**Tableau 4**

| Effet de l'AZT et du mimétique CD4M33 sur la réplication de l'isolat clinique VIH-1$_{LEN}$ dans les cultures de cellules mononucléées du sang périphérique (CMSP) humaines, activées par la phytohémoagglutinine-P (PHA-P). Les cellules ont été infectées par 50 TCID50 de virus : | | |
|---|---|---|
| VIH-1$_{LEN}$ | AZT | CD4M33 |
| DE$_{50}$ (nM) | 2,2 | 367 |
| DE$_{70}$ (nM) | 4,8 | 542 |
| DE$_{90}$ (nM) | 16,5 | 1006 |

## Exemple 8 : Exposition de sites antigéniques de l'enveloppe.

Afin d'évaluer la capacité du peptide de la présente invention CD4M33 à induire des variations conformationnelles dans la protéine gp120, caractérisée par l'exposition d'épitopes sensibles à des anticorps neutralisants, l'interaction de tels anticorps humains 48d et CG10 avec l'enveloppe VIH en présence du CD4M33 et, par comparaison, du CD4 recombinant, a été entreprise, en utilisant la technique de résonance plasmonique de surface. Les anticorps 48d et CG10 ont été fixés covalement à la surface des puces (bio-chip), puis des solutions de gp120, en absence de CD4 et en présence d'un excès de CD4 et de CD4M33 ont été injectées. Lorsque le CD4M33 est ajouté à la protéine virale gp120$_{HXB2,}$ avec un excès molaire de 1.5 et 20 fois, la protéine virale manifeste une forte interaction avec les anticorps (Figures 6a-b) ; par contre, en son absence (et en absence de CD4 soluble) la protéine de l'enveloppe interagit avec les anticorps très faiblement (Fig. 6a-b). En outre, l'effet de l'ajout de CD4M33 sur l'augmentation de l'affinité d'interaction de la protéine virale gp120 pour les anticorps est comparable à celui qui est obtenu par ajout de quantités équivalentes de CD4 soluble.

Les inventeurs ont conduit aussi des expériences similaires de résonance plasmonique en utilisant directement le VIH-1, souche HXB2. La figure 4 montre les résultats obtenus dans ces expériences et démontre l'évolution du signal de résonance plasmonique en fonction du temps après interaction d'une suspension de particules virales VIH-1$_{HXB2}$ avec une puce présentant les anticorps 48d et CG10 immobilisés : le VIH-1 interagit avec les anticorps seulement après incubation avec le peptide CD4M33 de la présente invention et, par contre, en son absence ou en présence du peptide [Ala23]CD4M9 inactif, l'interaction est pratiquement nulle.

La technique de résonance plasmonique de surface a donc permis de démontrer que la liaison du CD4M33 à la protéine de l'enveloppe gp120 est capable d'induire une modification de la protéine virale, qui, par suite, expose de façon préférentielle certaines surfaces moléculaires aux anticorps neutralisants (17b, CG10), isolés chez des personnes affectées par le VIH-1 [11], [12], [13].

L'exposition de ces épitopes cryptiques, induite par le peptide CD4M33 de la présente invention, est efficace tant dans la protéine de l'enveloppe sous forme recombinante que dans l'enveloppe des virions. De plus, aux vues des expériences récentes qui montrent que la protéine gp120 complexée au CD4 peut être une forme moléculaire capable d'induire la formation d'anticorps neutralisants [31, 32, 33] le complexe CD4M33-gp120 représente donc un candidat très intéressant en tant qu'immunogène pour l'induction d'anticorps neutralisants, notamment dans une application vaccinale.

Le CD4M33 possède la propriété de démasquer des epitopes de la protéine virale gp120, comme le fait le CD4 soluble, avec l'avantage qu'il ne pourra pas induire de réponse immune contre le CD4 et que, de plus, en raison de sa petite taille, il permettra un accès encore plus favorable aux épitopes démasqués de la protéine virale gp120.

## Exemple 9: Synthèse d'un support chromatographique pour la purification de l'enveloppe virale VIH.

Afin d'évaluer la possibilité d'utiliser le peptide de la présente invention mimétique du CD4 comme ligand immobilisé pour fonctionnaliser une matrice chromatographique pour la purification de la protéine virale gp120, un support polymérique hydrophile, le Sepharose 4B (Pharmacia, Uppsala, Suède) a été fonctionnalisé avec un de nos mimétiques CD4 et ensuite utilisé pour la fixation de la protéine virale gp120 directement à partir du milieu de culture de la protéine virale. Pour éviter d'utiliser des conditions trop drastiques lors de l'élution de la protéine gp120 du support et pour limiter donc les possibilités de dénaturation, un ligand à plus faible affinité pour la protéine virale gp120 a été choisi. Il s'agit du CD4M9 qui présente en ELISA une affinité (C1$_{50}$) pour la protéine virale gp120 entre 0.1 $\mu$M et 1.0 $\mu$M, selon l'isolat VIH-1. Le CD4M9 a été modifié sur la position Lysll, déjà utilisée dans les marquages précédents, pour inclure un groupement thiol supplémentaire (décrit dans l'exemple 1) qui a été ensuite utilisé pour son immobilisation covalente sur le support Sepharose 4B, comme décrit ci-dessous.

La résine EAH Sepharose (marque de commerce) (commercialisée par Pharmacia Biotech) comprenant 7 à 12 $\mu$moles de fonctions amines primaires par ml de résine sèche est fonctionnalisée par un groupement maléimide.

Pour cela, 12 ml de résine préalablement équilibrés dans 24 ml d'eau sont traités pendant 12h avec 1,05 mg de réactif sulfo SMCC (ester 3-sulfo-M-hydroxysuccinimide de l'acide (4-N-maléimido ethyl) cyclohexane-1-carboxylique (Sigma)) en maintenant le pH à environ 4,5. Le taux de substitution attendu est de 200 nmoles de fonction maléimide par ml de résine sèche. L'excès de réactif est ensuite retiré par lavages successifs avec 100 mM Tris-HCl, 500 mM NaCl, pH 8,0, puis avec un tampon 100 mM d'acétate sodique, 500 nM NaCl, pH 4,0. Les fonctions amines libres restantes sont ensuite acétylées par deux traitements avec une solution d'anhydride acétique à 1%. A la résine fonctionnalisée par le maléimide sont ajoutés 3,8 mg de peptide CD4M9 thiolé. La réaction est agitée à 4°C pendant 12 heures. La résine est ensuite lavée et équilibrée avec le tampon d'utilisation, 20 mM phosphate de sodium, 100 mM NaCl, pH 7,4. Une colonne chromatographique de 6.0 ml a été préparée avec cette matrice qui contient environ 0.1 mg/ml de peptide selon la présente invention CD4M9 immobilisé. Cette colonne a été ensuite chargée avec le milieu de culture de la gp120 et la protéine virale retenue sur la colonne a ensuite été éluée de la colonne avec traitement d'acide acétique 0.5 M. Les fractions éluées avec l'acide acétique ont été analysées par SDS-PAGE et ensuite lyophilisées. L'analyse de ces fractions a démontré qu'elles contenaient la protéine virale gp120.

La figure 8 est une représentation graphique du profil chromatographique obtenu dans cet exemple : absorbance à 280 nm (A280) en fonction du temps en minutes (t(min)).

Le profil chromatographique de la figure 8 montre plusieurs pics d'élution de la protéine de l'enveloppe. Les inventeurs pensent que les différents pics représentent la protéine virale gp120 à des degrés différents de dénaturation provoquée par l'acide acétique utilisé dans l'élution de la colonne.

## Exemple 10 : Criblage

Des molécules de la présente invention ont été marquées avec la fluorescéine ou la biotine sans altérer l'affinité de liaison à la protéine virale gp120.

Dans cet exemple, un marquage est effectué avec des métaux lanthanides fluorescents ou des isotopes radioactifs, sans altérer l'activité de liaison des peptides de la présente invention à la protéine virale gp120. Ces peptides marqués sont utilisés en tant que traceurs, dans des expériences de criblage par compétition de l'interaction de ces peptides avec la protéine virale gp120.

Dans une première expérience, la protéine virale gp120 est immobilisée dans des micro-plaques à 96 puits et le peptide CD4M33, marqué avec le lanthanide europium (III) est mis en présence de cette protéine dans des conditions permettant l'interaction entre le peptide de CD4M33 et la protéine gp120. Les mesures de signal (par exemple les mesures de fluorescence résolue dans le temps) permettent de quantifier la capacité des molécules criblées à déplacer les peptides marqués.

Dans une deuxième expérience, le peptide CD4M33, est marqué à la fluorescéine et lié à la protéine virale gp120, et est détecté par des mesures de polarisation de fluorescence en solution, dans des systèmes miniaturisés.

Ces deux systèmes, utilisés pour cribler des molécules capables d'inhiber l'interaction CD4-gp120, ne sont pas limitatifs. Les molécules détectées par ce criblage représentent des inhibiteurs de l'interaction CD4-gp120 et donc potentiellement des inhibiteurs de l'infection virale.

Les techniques de mise en oeuvre utilisées dans cet exemple sont décrites dans la littérature notamment dans les documents [37, 38, 39 et 40].

## REFERENCES

[1] Kwong, P.D., Wyatt, R., Robinson, J., Sweet, R.W., Sodroski, J., Hendrickson, W.A. (1998) Nature 393, 648-659

[2] Sweet, R.W., Truneh, A., Hendrickson, W.A. (1991) Curr. Opin. Biotechnol. 2, 622-633.

[3] Ryu, S.E., Trueh, A., Sweet, R.W., Hendrickson, W.A. (1994) Structure 2, 59-74.

[4] Vita, C., Drakopoulou, E., Vizzavona, J., Rochette, S., Martin, L., Ménez, A., Roumestand, C., Yang, Y.S., Ylistagui, L., Benjouad, A., Gluckman , J.C. 1999, Proc. Natl. Acad. Sci. USA, 96, 13091-13096

[5] Feng, Y., Broder, C.C., Kennedy, P.E., Berger, E.A.(1996) Science 272, 872-877.

[6] Choe, H., Farzan, M., Sun, Y., Sullivan, N., Rollins, B., Ponath, P.D., Wu, L., Mackay, C.R., LaRosa, G., Newman, W., Gerard, N., Gerard, C., Sodroski, J.(1996) Cell 85, 1135-1148.

[7] Dragic, T., Litwin, V., Allaway, G.P., Martin, S.R., Huang, Y., Nagashima, K.A., Cayanan, C., Maddon, P.J., Koup, R.A., Moore, J.P., Paxton, W.A. (1996) Nature 381, 667-673.

[8] Alkhatib, G., Combadiere. C., Broder, C.C., Feng,Y., Kennedy, P.E., Murphy, P.M., Berger, E.A. (1996) Science 272, 1955-1958.

[9] Wu, L., Gerard, N.P., Wyatt, R., Choe, H., Parolin, C., Ruffing, A., Cardoso, A.A., Desjardin, E., Newman, W., Gerard, C., Sodroski, J. (1996) Nature 384, 179-183.

[10] Trkola, A., Dragic, T., Arthos, J., Binley, J.M., Olson, W.C., Allaway, G.P., Cheng-Mayer, C., Robinson, J., Maddon, P.J., Moore, J.P. (1996) Nature 384, 184-187.

[11] Thali, M., Moore, J.P., Furman,. C., Charles, M., Ho, D.D., Robinson, J., Sodroski, J. (1993) J. Virol. 67, 3978-3988.

[12] Wyatt, R., Moore, J., Accola, M., Desjardin, E., Robinson, J., Sodroski, J. (1995) J. Virol. 69, 5723-5733.

[13] Moore, J., Sodroski J. (1996) J. Virol. 70, 1863-1872.

[14] Chen S., Chrusciel, R.A., Nakanishi, Ho, Raktabutr, A., Jonhson, M.E., Sato, A., Weiner, D., Hoxie, J., Sagarovi, H.U., Greene, M.I., Kahn, M. (1992), Proc. Natl. Acad. Sci., USA, 89, 5872-5876.

[15] Zhang, X., Gaubin, M., Briant, L., Srikantan, V., Murali, R., Saragoui, H.U., Weiner, D., Devaux, C., Autiero, M., Piatier-Tonneau, D., Greene, M.I. (1997), Nature Biotechnol., 15, 150-154

[16] Moore, J.P., Sweet, R.W. (1993), Perspect. Drug Disc. Design 1, 235-250.

[17] Allaway GP, Davis-Bruno KL, Beaudry GA, Garcia EB, Wong EL, Ryder AM, Hasel KW, Gauduin MC, Koup RA, McDougal JS, et al.. (1995) AIDS Res Hum Retroviruses. 11, 533-539

[18] Otomo, H., Wesolowski, J., Way, J.C., Itoh, I., Ono, M., Wada, Y., Wu, Y., Nemori, R., Jinbo, Y., Wang, H., Lo, K.M., Yamaguchi, N., Brunkhorst, B. Gillies, S., Chen, L.B. (1997) Nat.Biotechnol. 15, 343-348.

[19] Ojwang, J.O., Buckheit, R.W., Pommier, Y., Mazumder, A., De Vreese, K., Este, J.A., Reymen, D., Pallansch, L.A., Lackman-Smith, C., Wallace, T.L., et al. (1995) Antimicrob. Agents Chemother. 39, 2426-2435.

[20] Rusconi, S., Moonis, M., Merrill, D.P., Pallai, P.V., Neidhardt, E.A., Singh, S.K., Willis, K.J., Osburne, M.S., Profy, A.T., Jenson, J.C., Hirsch, M.S. (1996) Antimicrob. Agents Chemother. 40, 234-236.

[21] De Clercq, E. (1998) Pure Appl. Chem. 70, 567-577.

[22] Yahi, N., Fantini, J., Baghdiguian, S., Mabrouk, K., Tamalet, C:, Rochat, H., Van Rietschoten, J., Sabatier, J.M. (1995) Proc. Natl. Acad. Sci. USA 92, 4867-4871.

[23] De Clercq, E., Yamamoto, N., Pauwels, R., Balzarini, J., Witvrouw, M., De. Vreese, K., Debyser, Z., Rosenwirth, B., Peichl, P., Datema, R., Thornton, D., Skerlj, R., Gaul, F., Padmanabhan, S., Bridger, G., Henson, G., Abrams, M. (1994) Antimicrob. Agents Chemother. 38, 668-674.

[24] Howard, O.M., Oppenheim, J.J., Hollingshead, M.G., Covey, J.M., Bigelow, J., McCormack, J.J., Buckheit, R.W. Jr, Clanton, D.J., Turpin, J.A., Rice, W.G. (1998) J. Med. Chem. 41, 2184-2193.

[25] Tamamura, H., Muratami, T. Masuda, M., Otaka, A., Takeda, W., Ibuka, T., Nakashira, H., Waki, M., Matsumoto A., Yamamoto, N. et al. (1994), Biochem. Biophys. Res. Commun, 205, 1729-1735.

[26] Tamamura, H., Omagari, A., Oishi, S., Kanamoto, T., Yamamoto, N., Peiper, S.C., Nakasima, H., Otaka, A., Pujiri, N. (2000) Bioorg. Med. Chem. Lett. 10, 2633-2637.

[27] Baba, M., Nishimura, O., Kanzaki, N., Okamoto, M., Sawada, H., Iizawa, Y., Shiraishi, M., Aramaki, Y., Okonogi, K., Ogawa, Y., Meguro, K., Fujino, M. (1999) Proc. Natl. Acad. Sci. USA 96, 5698-5703.

[28] Wild, C., Dubay, J.W., Greenwell, T., Baird, T. Jr, Oas, T.G., McDanal, C., Hunter, E., Matthews, T. (1994) Proc. Natl. Acad. Sci. USA 91, 9770-9774.

[29] Rimsky, L.T., Shugars, D.C., Matthews, T.J. (1998) J. Virol. 72, 986-993.

[30] Chun, T.W, Fauci, A.S. (1999) Proc. Natl. Acad. Sci. USA 96, 10958-10961.

[31] LaCasse R.A., Follis K.E., Trahey, M., Scarborough, J.D., Littman, D.R., Nunberg, J.H. (1999) Science 283, 357-362.

[32] Devico A, Silver A, Thronton AM, Sarngadharan MG, Pal R. (1996) Virology 218, 258-63.

[33] Fouts TR, Tuskan R, Godfrey K, Reitz M, Hone D, Lewis GK, DeVico AL. (2000) J Virol 74, 11427-36

[34] Rossio, J.L., Esser, M.T., Suryanarayana, K., Schneider, D.K., Bess, J.W. Jr, Vasquez, G.M., Wiltrout, T.A., Chertova, E., Grimes, M.K., Sattentau, Q., Arthur, L.O., Henderson, L.E., Lifson, J.D. (1998) J. Virol. 72, 7992-8001.

[35] Lee, S., Peden, K. Dimitrov, D.S., Broder, C.C., Manischewitz, J., Denisova, G., Gershoni, J.M., Golding, H. (1997) J. Virol. 71, 6037-6043.

[36] Wu, M., Myszka, D.G., Tendian, S.W., Brouillette, C.G., Sweet, R.W., Chaiken, I.M., Heudrickson, W.A. (1996), Proc. Natl. Acad. Sci. USA 93, 120530-15035.

[37] McMahon J.B., Beutler, J.A., O'Keefe, B.R., Goodrum, C.B., Myers, M.A., Boyd, M.R. (2000) J. Biomol. Screen 5, 169-176.

[38] Sportman, J.R., Leytes, L.J. (2000) Drug Discov, Today 1, 27-32.

[39] Mathis G., Biochemistry, 1993, 32(43), 11682-7.

[40] Pernelle C., et al.; Clin. Chem., 1993, 39(9), 1953-9.

## Revendications

1.  Peptide **caractérisé en ce qu'**il comprend la séquence (I) suivante :

    TPA- Xaa$^a$- Xaa$^b$- Ala ou Gln ou His - Arg ou Phe - Cys - xaa$^c$- Xaa$^d$- Arg- Cys - Lys - Xaa$^e$- Xaa$^f$-Xaa$^g$- Xaa$^h$- Leu ou Lys - Xaa$^i$ - Lys - Cys - Ala ou Gln - Gly ou (D)Asp ou Ser - Ser ou His ou Asn-Xaa$^j$- Cys - Thr ou Ala - Cys - Xaa$^k$-NH$_2$,

    dans laquelle TPA représente l'acide thiopropionique, Xaa$^a$, Xaa$^b$, Xaa$^c$, Xaa$^d$, Xaa$^e$, Xaa$^f$, Xaa$^g$, Xaa$^h$, et Xaa$^i$, sont des acides aminés, naturels ou non naturels, identiques ou différents, Xaa$^j$ représente la β-naphtylalanine ou la phénylalanine ou la bi-phénylalanine, et Xaa$^k$ représente Gly ou Val ou Ile.

2.  Peptide selon la revendication 1, dans lequel la séquence (I) peut comprendre en outre un résidu proline lié au résidu Xaa$^k$.

3.  Peptide selon la revendication 1 ou 2, dans lequel Xaa$^i$ est Gly.

4.  Peptide comprenant une séquence choisie parmi les séquences ID n°4, ID n°5, ID n°6, ID n°7, ID n°8, ID n°9, ID n° 10, ID n°11, ID n°12, ID n°13, ID n°14, ID n°15 et ID n°16 de la liste de séquence annexée.

5. Procédé de fabrication d'un peptide selon l'une quelconque des revendications 1 à 4, ledit procédé comprenant une synthèse chimique en phase solide dudit peptide.

6. Utilisation d'un peptide selon l'une quelconque des revendications 1 à 4 pour préparer un médicament.

7. Utilisation d'un peptide selon l'une quelconque des revendications 1 à 4 pour préparer un agent antiviral.

8. Utilisation d'un peptide selon l'une quelconque des revendications 1 à 4 pour préparer un médicament destiné au traitement du SIDA.

9. Vaccin comprenant un complexe d'un peptide selon l'une quelconque des revendications 1 à 4 et d'une protéine d'enveloppe d'un virus.

10. Vaccin selon la revendication 9, dans lequel le virus est un virus du SIDA.

11. Utilisation in vitro d'un peptide selon l'une quelconque des revendications 1 à 4 pour la détection de molécules de l'enveloppe virale du VIH.

12. Utilisation d'un peptide selon l'une quelconque des revendications 1 à 4 pour fabriquer un produit de diagnostic.

13. Utilisation d'un peptide selon l'une quelconque des revendications 1 à 4 comme ligand immobilisé pour fonctionnaliser une matrice de chromatographie.

14. Utilisation in vitro d'un peptide selon l'une quelconque des revendications 1 à 4 dans un procédé de criblage.

**Claims**

1. Peptide **characterized in that** it comprises the following sequence (I):

    TPA- $Xaa^a$- $Xaa^b$- Ala or Gln or His - Arg or Phe -Cys - $Xaa^c$- $Xaa^d$- Arg - Cys - Lys - $Xaa^e$- $Xaa^f$-$Xaa^g$-$Xaa^h$-Leu or Lys - $Xaa^i$ - Lys - Cys - Ala or Gln-Gly or (D)Asp or Ser - Ser or His or Asn - $Xaa^j$- Cys-Thr or Ala - Cys - $Xaa^k$-NH$_2$,

    in which TPA represents thiopropionic acid, $Xaa^a$, $Xaa^b$, $Xaa^c$, $Xaa^d$, $Xaa^e$, $Xaa^f$, $Xaa^g$, $Xaa^h$ and $Xaa^i$ are natural or unnatural, identical or different amino acids, $Xaa^j$ represents β-naphthylalanine or phenylalanine or diphenylalanine, and $Xaa^k$ represents Gly or Val or Ile.

2. Peptide according to Claim 1, in which the sequence (I) may also comprise a proline residue linked to the residue $Xaa^k$.

3. Peptide according to Claim 1 or 2, in which $Xaa^i$ is Gly.

4. Peptide comprising a sequence chosen from sequences ID No. 4, ID No. 5, ID No. 6, ID No. 7, ID No. 8, ID No. 9, ID No. 10, ID No. 11, ID No. 12, ID No. 13, ID No. 14, ID No. 15 and ID No. 16 of the attached sequence listing.

5. Method for producing a peptide according to any one of Claims 1 to 4, said method comprising solid-phase chemical synthesis of said peptide.

6. Use of a peptide according to any one of Claims 1 to 4, for preparing a medicinal product.

7. Use of a peptide according to any one of Claims 1 to 4, for preparing an antiviral agent.

8. Use of a peptide according to any one of Claims 1 to 4, for preparing a medicinal product intended for the treatment of AIDS.

9. Vaccine comprising a complex of a peptide according to any one of Claims 1 to 4 and an envelope protein of a virus.

10. Vaccine according to Claim 9, in which the virus is an AIDS virus.

11. In vitro use of a peptide according to any one of Claims 1 to 4, for detecting molecules of the viral envelope of HIV.

12. Use of a peptide according to any one of Claims 1 to 4, for producing a diagnostic product.

13. Use of a peptide according to any one of Claims 1 to 4, as an immobilized ligand for functionalizing a chromatographic matrix.

14. In vitro use of a peptide according to any one of Claims 1 to 4, in a screening method.


**Patentansprüche**

1. Peptid, **dadurch gekennzeichnet, daß** es die folgende Sequenz (I) umfaßt:

TPA- $Xaa^a$- $Xaa^b$- Ala oder Gln oder His - Arg oder Phe-Cys - $Xaa^c$- $Xaa^d$- Arg- Cys - Lys - $Xaa^e$- $Xaa^f$-$Xaa^g$-$Xaa^h$- Leu oder Lys - $Xaa^i$ - Lys - Cys - Ala oder Gln - Gly oder (D)Asp oder Ser - Ser oder His oder Asn-$Xaa^j$ - Cys - Thr oder Ala - Cys - $Xaa^k$ -$NH_2$,

worin TPA Thiopropionsäure darstellt, $Xaa^a$, $Xaa^b$, $Xaa^c$, $Xaa^d$, $Xaa^e$, $Xaa^f$, $Xaa^g$, $Xaa^h$ und $Xaa^i$ natürliche oder nicht-natürliche, gleiche oder verschiedene Aminosäuren sind, $Xaa^j$ β-Naphthylalanin oder Phenylalanin oder Diphenylalanin darstellt und $Xaa^k$ Gly oder Val oder Ile darstellt.

2. Peptid gemäß Anspruch 1, wobei Sequenz (I) weiter einen mit dem Rest $Xaa^k$ verbundenen Prolinrest umfassen kann.

3. Peptid gemäß Anspruch 1, worin $Xaa^i$ Gly ist.

4. Peptid umfassend eine Sequenz, die aus den Sequenzen ID Nr. 4, ID Nr. 5, ID Nr. 6, ID Nr. 7, ID Nr. 8, ID Nr. 9, ID Nr. 10, ID Nr. 11, ID Nr. 12, ID Nr. 13, ID Nr. 14, ID Nr. 15 und ID Nr. 16 der beigefügten Sequenzliste ausgewählt ist.

5. Verfahren zur Herstellung eines Peptids gemäß einem der Ansprüche 1 bis 4, wobei das Verfahren eine chemische Festphasensynthese des Peptids umfaßt.

6. Verwendung eines Peptids gemäß einem der Ansprüche 1 bis 4 zum Herstellen eines Arzneimittels.

7. Verwendung eines Peptids gemäß einem der Ansprüche 1 bis 4 zum Herstellen eines antiviralen Mittels.

8. Verwendung eines Peptids gemäß einem der Ansprüche 1 bis 4 zum Herstellen eines zur Behandlung von AIDS bestimmten Mittels.

9. Vakzin umfassend einen Komplex aus einem Peptid gemäß einem der Ansprüche 1 bis 4 und einem Hüllprotein eines Virus.

10. Vakzin gemäß Anspruch 9, wobei das Virus ein AIDS-Virus ist.

11. Verwendung in vitro eines Peptids gemäß einem der Ansprüche 1 bis 4 zum Nachweis von Molekülen der Virushülle des HIV.

12. Verwendung eines Peptids gemäß einem der Ansprüche 1 bis 4 zum Herstellen eines diagnostischen Produkts.

13. Verwendung eines Peptids gemäß einem der Ansprüche 1 bis 4 als immobilisierter Ligand zum Funktionalisieren einer chromatographischen Matrix.

14. Verwendung in vitro eines Peptids gemäß einem der Ansprüche 1 bis 4 bei einem Durchmusterungsverfahren.

−◇−CD4M9 −□−CD4M9−T −△−CD4M27 −✳−CD4M32 −✧−CD4M33 −●−CD4M35

## Fig. 1

−◇−CD4M9 −✧−CD4M33 −●−CD4M35 −✳−CD4M33−F

## Fig. 2

**Fig. 3**

**Fig. 6A**

**Fig. 6B**

—✕—gp120+CD4M27    —▫—gp120+CD4M9    —△—gp120+(A23)CD4M9

—●—gp120 seule    —gp120+CD4s

—□—pas de peptide

—△—[Ala23] CD4M9 (10μg/ml)

—⊖—CD4M33 (10μg/ml)

Fig. 4

EP 1 368 372 B1

**Fig. 5A**

**Fig. 5B**

**Fig. 5C**

```
                                    Q I K I L G N Q G S F L T K G P    sCDA
                                    33                            48   (CDR2)

    Z F T N V S C T T S   K E C W S V C Q R L H N T S R G K C M N K K C R C Y S    ChTX
    1       10                        20                  30            37

    - - - - V S C T T S   K E C W S V C Q R L H N T S K G G C Q G S F C T C G P    CD4M

          A F C   N L R M C Q L S C R S L G L L G K C I G D K C E C V K H         ScTx
          1                                                        31

          - - C   N L A R C Q L S C K S L G L K G G C Q G S F C T C G - -         CD4M3
              1                                                27

          - - C   N L A R C Q L R C K S L G L L G K C A G S F C A C G P -         CD4M9
          - - TPA N L A R C Q L R C K S L G L L G K C A G S F C A C G P -         CD4M9T
          - - TPA N L A F C Q L R C K S L G L L G K C A G S F C A C V - -         CD4M27
          - - TPA N L A F C Q L R C K S L G L L G K C A S S F C A C V - -         CD4M27A
          - - TPA N L A F C Q L R C K S L G L L G K C A G H F C A C V - -         CD4M27B
          - - TPA N L A F C Q L R C K S L G L L G K C A G N F C A C V - -         CD4M27C
          - - TPA N L Q F C Q L R C K S L G L L G K C A G S B C A C V - -         CD4M30
          - - TPA N L A R C Q L R C K S L G L L G K C A G S B C A C V - -         CD4M32
          - - TPA N L H F C Q L R C K S L G L L G K C A G S B C A C V - -         CD4M33
          - - TPA N L H F C Q L R C K S L G L L G K C A d S B C A C I - -         CD4M35
          - - TPA N L H F C V Q R C H S L G K L G K C A G S F C A C V - -         K15
          - - TPA N L H F C V Q R C H S L G L K G K C A G S F C A C V - -         K16
```

## Fig. 7

EP 1 368 372 B1

**Fig. 8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4721704 A, CHANG JAW-KANG
- US 4684622 A, HUFFMAN WILLIAM F
- EP 0989136 A

**Littérature non-brevet citée dans la description**

- **J.H. NUNBERG.** Montana Biotechnology Center. The University of Montana
- **A. DEVICO.** Institute of Human Virology. University of Maryland
- **MARTIN et al.** Engineering Novel Bioactive Mini-Proteins on Natural Scaffolds. *TETRAHEDRON,* 24 Novembre 2000, vol. 56, 9451-9460
- **H. OZAKI et al.** Molecular Structure of the Toxic Domain of Heat-Stable Enterotoxin Produced by a Pathogenic Strain of Escherichia coli. *THE JOURNAL OF BIOLOGICAL CHEMISTRY,* 25 Mars 1991, vol. 266, 5934-5941
- **SOMBROOK ; FRITSCH ; MANIATIS.** MOLECULAR CLONING, A LABORATORY MANUAL
- **KWONG, P.D ; WYATT, R ; ROBINSON, J ; SWEET, R.W ; SODROSKI, J ; HENDRICKSON, W.A.** *Nature,* 1998, vol. 393, 648-659
- **SWEET, R.W ; TRUNEH, A ; HENDRICKSON, W.A.** *Curr. Opin. Biotechnol.,* 1991, vol. 2, 622-633
- **RYU, S.E ; TRUEH, A ; SWEET, R.W ; HENDRICKSON, W.A.** *Structure,* 1994, vol. 2, 59-74
- **VITA, C ; DRAKOPOULOU, E ; VIZZAVONA, J ; ROCHETTE, S ; MARTIN, L ; MÉNEZ, A ; ROUMESTAND, C ; YANG, Y.S ; YLISTAGUI, L ; BENJOUAD, A.** *Proc. Natl. Acad. Sci. USA,* 1999, vol. 96, 13091-13096
- **FENG, Y ; BRODER, C.C ; KENNEDY, P.E ; BERGER, E.A.** *Science,* 1996, vol. 272, 872-877
- **CHOE, H ; FARZAN, M ; SUN, Y ; SULLIVAN, N ; ROLLINS, B ; PONATH, P.D ; WU, L ; MACKAY, C.R ; LAROSA, G ; NEWMAN, W.** *Cell,* 1996, vol. 85, 1135-1148
- **DRAGIC, T ; LITWIN, V ; ALLAWAY, G.P ; MARTIN, S.R ; HUANG, Y ; NAGASHIMA, K.A ; CAYANAN, C ; MADDON, P.J ; KOUP, R.A ; MOORE, J.P.** *Nature,* 1996, vol. 381, 667-673
- **ALKHATIB, G ; COMBADIERE. C ; BRODER, C.C ; FENG,Y ; KENNEDY, P.E ; MURPHY, P.M ; BERGER, E.A.** *Science,* 1996, vol. 272, 1955-1958
- **WU, L ; GERARD, N.P ; WYATT, R ; CHOE, H ; PAROLIN, C ; RUFFING, A ; CARDOSO, A.A ; DESJARDIN, E ; NEWMAN, W ; GERARD, C.** *Nature,* 1996, vol. 384, 179-183
- **TRKOLA, A ; DRAGIC, T ; ARTHOS, J ; BINLEY, J.M ; OLSON, W.C ; ALLAWAY, G.P ; CHENG-MAYER, C ; ROBINSON, J ; MADDON, P.J ; MOORE, J.P.** *Nature,* 1996, vol. 384, 184-187
- **THALI, M ; MOORE, J.P ; FURMAN,. C ; CHARLES, M. ; HO, D.D ; ROBINSON, J ; SODROSKI, J.** *J. Virol.,* 1993, vol. 67, 3978-3988
- **WYATT, R ; MOORE, J ; ACCOLA, M ; DESJARDIN, E ; ROBINSON, J ; SODROSKI, J.** *J. Virol,* 1995, vol. 69, 5723-5733
- **MOORE, J ; SODROSKI J.** *J. Virol,* 1996, vol. 70, 1863-1872
- **CHEN S ; CHRUSCIEL, R.A ; NAKANISHI, HO ; RAKTABUTR, A ; JONHSON, M.E ; SATO, A ; WEINER, D ; HOXIE, J ; SAGAROVI, H.U ; GREENE, M.I.** *Proc. Natl. Acad. Sci.,* 1992, vol. 89, 5872-5876
- **ZHANG, X ; GAUBIN, M ; BRIANT, L ; SRIKANTAN, V ; MURALI, R ; SARAGOUI, H.U ; WEINER, D ; DEVAUX, C ; AUTIERO, M ; PIATIER-TONNEAU, D.** *Nature Biotechnol.,* 1997, vol. 15, 150-154
- **MOORE, J.P ; SWEET, R.W.** *Perspect. Drug Disc. Design,* 1993, vol. 1, 235-250
- **ALLAWAY GP ; DAVIS-BRUNO KL ; BEAUDRY GA ; GARCIA EB ; WONG EL ; RYDER AM ; HASEL KW ; GAUDIN MC ; KOUP RA ; MCDOUGAL JS et al.** *AIDS Res Hum Retroviruses,* 1995, vol. 11, 533-539
- **OTOMO, H ; WESOLOWSKI, J ; WAY, J.C ; ITOH, I ; ONO, M ; WADA, Y ; WU, Y ; NEMORI, R ; JINBO, Y ; WANG, H.** *Nat.Biotechnol.,* 1997, vol. 15, 343-348
- **OJWANG, J.O ; BUCKHEIT, R.W ; POMMIER, Y ; MAZUMDER, A ; DE VREESE, K ; ESTE, J.A ; REYMEN, D ; PALLANSCH, L.A ; LACKMAN-SMITH, C ; WALLACE, T.L et al.** *Antimicrob. Agents Chemother.,* 1995, vol. 39, 2426-2435

- **RUSCONI, S ; MOONIS, M ; MERRILL, D.P ; PALLAI, P.V ; NEIDHARDT, E.A ; SINGH, S.K ; WILLIS, K.J ; OSBURNE, M.S ; PROFY, A.T ; JENSON, J.C.** *Antimicrob. Agents Chemother.,* 1996, vol. 40, 234-236
- **DE CLERCQ, E.** *Pure Appl. Chem.,* 1998, vol. 70, 567-577
- **YAHI, N ; FANTINI, J ; BAGHDIGUIAN, S ; MABROUK, K ; TAMALET, C ; ROCHAT, H ; VAN RIETSCHOTEN, J ; SABATIER, J.M.** *Proc. Natl. Acad. Sci. USA,* 1995, vol. 92, 4867-4871
- **DE CLERCQ, E ; YAMAMOTO, N ; PAUWELS, R ; BALZARINI, J ; WITVROUW, M ; DE. VREESE, K ; DEBYSER, Z ; ROSENWIRTH, B ; PEICHL, P ; DATEMA, R.** *Antimicrob. Agents Chemother.,* 1994, vol. 38, 668-674
- **HOWARD, O.M ; OPPENHEIM, J.J ; HOLLINGSHEAD, M.G ; COVEY, J.M ; BIGELOW, J ; MCCORMACK, J.J ; BUCKHEIT, R.W. JR ; CLANTON, D.J ; TURPIN, J.A ; RICE, W.G.** *J. Med. Chem.,* 1998, vol. 41, 2184-2193
- **TAMAMURA, H ; MURATAMI, T ; MASUDA, M ; OTAKA, A ; TAKEDA, W ; IBUKA, T ; NAKASHIRA, H ; WAKI, M ; MATSUMOTO A ; YAMAMOTO, N et al.** *Biochem. Biophys. Res. Commun,* 1994, vol. 205, 1729-1735
- **TAMAMURA, H ; OMAGARI, A ; OISHI, S ; KANAMOTO, T ; YAMAMOTO, N ; PEIPER, S.C ; NAKASIMA, H ; OTAKA, A ; PUJIRI, N.** *Bioorg. Med. Chem. Lett,* 2000, vol. 10, 2633-2637
- **BABA, M ; NISHIMURA, O ; KANZAKI, N ; OKAMOTO, M ; SAWADA, H ; IIZAWA, Y ; SHIRAISHI, M ; ARAMAKI, Y ; OKONOGI, K ; OGAWA, Y.** *Proc. Natl. Acad. Sci. USA,* 1999, vol. 96, 5698-5703
- **WILD, C ; DUBAY, J.W ; GREENWELL, T ; BAIRD, T. JR ; OAS, T.G ; MCDANAL, C ; HUNTER, E ; MATTHEWS, T.** *Proc. Natl. Acad. Sci. USA,* 1994, vol. 91, 9770-9774
- **RIMSKY, L.T ; SHUGARS, D.C ; MATTHEWS, T.J.** *J. Virol.,* 1998, vol. 72, 986-993
- **CHUN, T.W ; FAUCI, A.S.** *Proc. Natl. Acad. Sci. USA,* 1999, vol. 96, 10958-10961
- **LACASSE R.A ; FOLLIS K.E ; TRAHEY, M ; SCARBOROUGH, J.D ; LITTMAN, D.R ; NUNBERG, J.H.** *Science,* 1999, vol. 283, 357-362
- **DEVICO A ; SILVER A ; THRONTON AM ; SARNGADHARAN MG ; PAL R.** *Virology,* 1996, vol. 218, 258-63
- **FOUTS TR ; TUSKAN R ; GODFREY K ; REITZ M ; HONE D ; LEWIS GK ; DEVICO AL.** *J Virol.,* 2000, vol. 74, 11427-36
- **ROSSIO, J.L ; ESSER, M.T ; SURYANARAYANA, K ; SCHNEIDER, D.K ; BESS, J.W. JR ; VASQUEZ, G.M ; WILTROUT, T.A ; CHERTOVA, E ; GRIMES, M.K ; SATTENTAU, Q.** *J. Virol,* 1998, vol. 72, 7992-8001
- **LEE, S ; PEDEN, K ; DIMITROV, D.S ; BRODER, C.C ; MANISCHEWITZ, J ; DENISOVA, G ; GERSHONI, J.M ; GOLDING, H.** *J. Virol.,* 1997, vol. 71, 6037-6043
- **WU, M ; MYSZKA, D.G. ; TENDIAN, S.W ; BROUILLETTE, C.G ; SWEET, R.W ; CHAIKEN, I.M ; HEUDRICKSON, W.A.** *Proc. Natl. Acad. Sci. USA,* 1996, vol. 93, 120530-15035
- **MCMAHON J.B ; BEUTLER, J.A ; O'KEEFE, B.R ; GOODRUM, C.B ; MYERS, M.A ; BOYD, M.R.** *J. Biomol. Screen,* 2000, vol. 5, 169-176
- **SPORTMAN, J.R ; LEYTES, L.J.** *Drug Discov, Today,* 2000, vol. 1, 27-32
- **MATHIS G.** *Biochemistry,* 1993, vol. 32 (43), 11682-7
- **PERNELLE C. et al.** *Clin. Chem.,* 1993, vol. 39 (9), 1953-9